# EUROPEAN PATENT APPLICATION

(11) **EP 4 006 051 A1**
(43) Date of publication of application: **01.06.2022**
(21) Application number: 21189336.7
(22) Date of filing: 08.03.2016
(51) Int. Cl.: C07K 16/00, A61K 38/17, A61K 45/06, C07K 16/28, C07K 16/40, C07K 14/47, A61K 39/00, A61P 21/04, A61P 25/00, A61P 25/08, A61P 43/00, A61P 7/00, A61P 37/06

(54) **METHODS OF REDUCING SERUM LEVELS OF FC-CONTAINING AGENTS USING FCRN ANTAGONISTS**

(30) Priority: 09.03.2015 US 201562130076 P
(62) Divisional of application: 16719896.9
(71) Applicant: Argenx BVBA, 9052 Zwijnaarde (BE)
(72) Inventor: ONGENAE, Nicolas, G.h, 4300 Waremme (BE); DREIER, Torsten, 9830 Sint Martens Latern (BE); ULRICHTS, Peter, 9070 Destelbergen (BE); DE HAARD, Johannes, 4436 Oudelande (NL); BLANCHETOT, Christophe, 9070 Destelbergen (BE)
(74) Representative: Boult Wade Tennant LLP

(57) **Abstract**

Provided are novel methods of reducing the serum levels of Fc-containing agents (e.g., antibodies and immunoadhesins) in a subject. These methods generally comprise administering to the subject an effective amount of an isolated FcRn-antagonist that binds specifically to FcRn with increased affinity and reduced pH dependence relative to the native Fc region. The disclosed methods are particularly useful for treating antibody-mediated disorders (e.g. autoimmune diseases).

## Description

### RELATED APPLICATIONS

This application clauses the benefit of U.S. Provisional Application No.: 62/130,076, filed March 9, 2015, which is hereby incorporated by reference in its entirety.

### BACKGROUND

Immunoglobulin gamma (IgG) antibodies play a key role in the pathology of many disorders, such as autoimmune diseases, inflammatory diseases, and disorders in which the pathology is characterized by over-expression of IgG antibodies (e.g., hypergammaglobulinemia) (see e.g. Junghans, Immunologic Research 16 (1):29 (1997)).

The half-life of IgG in the serum is prolonged relative to the serum half-life of other plasma proteins (Roopenian et al., J. Immunology 170:3528 (2003); Junghans and Anderson, Proc. Natl. Acad. Sci. USA 93:5512 (1996)). This long half-life is due, in part, to the binding of the Fc region of IgG to the Fc receptor, FcRn. Although FcRn was originally characterized as a neonatal transport receptor for maternal IgG, it also functions in adults to protect IgG from degradation. FcRn binds to pinocytosed IgG and protects the IgG from transport to degradative lysosomes by recycling it back to the extracellular compartment. This recycling is facilitated by the pH dependent binding of IgG to FcRn, where the IgG/FcRn interaction is stronger at acidic endosomal pH than at extracellular physiological pH.

When the serum concentration of IgG reaches a level that exceeds available FcRn molecules, unbound IgG is not protected from degradative mechanisms and will consequently have a reduced serum half-life. Thus, inhibition of IgG binding to FcRn reduces the serum half-life of IgG by preventing IgG endosomal recycling of IgG. Accordingly, agents that antagonize the binding of IgG to FcRn may be useful for regulating, treating or preventing antibody-mediated disorders, such as autoimmune diseases, inflammatory diseases, etc. One example of a method of antagonzing IgG Fc binding to FcRn involves the generation of blocking antibodies to FcRn (see e.g WO2002/43658). Peptides have also been identified that bind to and antagonize FcRn function (see e.g. US6,212,022 and US8,101,186). In addition, full-length IgG antibodies comprising variant Fc receptors with enhanced FcRn binding and decreased pH dependence have also been identified that antagonize FcRn binding to IgG (see e.g. 8,163,881). However, there is a need in the art for improved methods for the treatment of antibody-mediated disorders.

### SUMMARY

The present disclosure provides novel methods of reducing the serum levels of Fc-containing agents (e.g., antibodies and immunoadhesins) in a subject. These methods generally comprise administering to the subject an effective amount of an isolated FcRn-antagonist that binds specifically to FcRn with increased affinity and reduced pH dependence relative to the native Fc region. The disclosed methods are particularly useful for treating antibody-mediated disorders (e.g. autoimmune diseases).

Accordingly, in one aspect, the instant disclosure provides a method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject in a dose of between about 0.2 and about 200 mg/kg.

In another aspect, the instant disclosure provides a method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject at least twice in 20 days.

The following embodiments apply to all aspects of the instant disclosure.

In certain embodiments, the FcRn-antagonist is administered to the subject at a frequency of once every 1, 2, 3, 4, 5, 6, 7, 8, 9 or 10 days. In certain embodiments, the FcRn-antagonist is administered to the subject at a frequency of once every 4 days. In certain embodiments, the FcRn-antagonist is administered to the subject at a frequency of once every 7 days. In certain embodiments, the FcRn-antagonist is administered to the subject 2, 3, 4, 5, 6, 7, 8, 9, 10, 11, 12, 13, 14, 15, 16, 17, 18, 19, or 20 times in 20 days.

In another aspect, the instant disclosure provides a method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject at a frequency of once every 48 hours for four weeks.

In certain embodiments, the FcRn-antagonist is administered to the subject in a dose of between about 0.2 and about 200 mg/kg. In certain embodiments, the FcRn-antagonist is administered to the subject in a dose of about 0.2, 1, 2, 3, 5, 10, 20, 25, 30, 50, 70, 100, or 200 mg/kg. In certain embodiments, the FcRn-antagonist is administered to the subject in a dose of about 10 mg/kg. In certain embodiments, the FcRn-antagonist is administered to the subject in a dose of about 20 mg/kg. In certain embodiments, the FcRn-antagonist is administered to the subject in a dose of about 25 mg/kg.

In another aspect, the instant disclosure provides a method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject in a dose of about 25 mg/kg at a frequency of once every four days.

In another aspect, the instant disclosure provides a method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject in a dose of about 25 mg/kg at a frequency of once every seven days.

In certain embodiments, the FcRn-antagonist is administered intravenously. In certain embodiments, the FcRn-antagonist is administered subcutaneously. In certain embodiments, the FcRn-antagonist is administered to the subject in more than one dose, wherein the first dose administered to the subject is administered intravenously, and wherein one or more of the second or subsequent doses are administered subcutaneously.

In certain embodiments, the FcRn-antagonist does not comprise an antibody variable region. In certain embodiments, the FcRn-antagonist does not comprise a CH1 domain. In certain embodiments, the FcRn-antagonist does not comprise a free cysteine residue. In certain embodiments, the variant Fc region is an IgG Fc region. In certain embodiments, the variant Fc region is an IgG1 Fc region. In certain embodiments, the amino acid sequence of the Fc domains of the variant Fc region comprises the amino acid sequence set forth in SEQ ID NO:1, 2, or 3. In certain embodiments, the amino acid sequence of the Fc domains of the variant Fc region consists of the amino acid sequence set forth in SEQ ID NO:1. In certain embodiments, the FcRn-antagonist consists of a variant Fc region, wherein the amino acid sequence of the Fc domains of the variant Fc region consists of the amino acid sequence set forth in SEQ ID NO:1, 2, or 3.

In certain embodiments, the variant Fc region has an increased affinity for an Fc gamma receptor relative to the affinity of a wild-type IgG1 Fc region for the Fc gamma receptor. In certain embodiments, the variant Fc region has increased affinity for CD16a. In certain embodiments, the Fc domains of the variant Fc region do not comprise an N-linked glycan at EU position 297. In certain embodiments, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region, or FcRn-binding fragment thereof, comprising an afucosylated N-linked glycan at EU position 297. In certain embodiments, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region or FcRn-binding fragment thereof, comprising an N-linked glycan having a bisecting GlcNac at EU position 297.

In certain embodiments, the variant Fc region is linked to a half-life extender. In certain embodiments, the half-life extender is polyethylene glycol or human serum albumin.

In certain embodiments, the Fc-containing agent is an antibody or immunoadhesin. In certain embodiments, the Fc-containing agent is a therapeutic or diagnostic agent. In certain embodiments, the Fc-containing agent is an imaging agent. In certain embodiments, the Fc-containing agent is an antibody drug conjugate. In certain embodiments, the Fc-containing agent is a pathogenic antibody. In certain embodiments, the Fc-containing agent is an autoantibody.

In certain embodiments, the subject has an antibody-mediated disease or disorder, wherein administration of the FcRn-antagonist to the subject ameliorates the disease or disorder. In certain embodiments, the disease or disorder is treatable using intravenous immunoglobulin (IVIG), plasmapheresis and/or immunoadsorption. In certain embodiments, the antibody-mediated disease or disorder is an autoimmune disease. In certain embodiments, the autoimmune disease is selected from the group consisting of allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, dilated cardiomyopathy, discoid lupus, epidermolysis bullosa acquisita, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic membranous neuropathy, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lichen sclerosus, lupus erthematosis, Meniere's disease, mixed connective tissue disease, mucous membrane pemphigoid, multiple sclerosis, type 1 diabetes mellitus, Multifocal motor neuropathy (MMN), myasthenia gravis, paraneoplastic bullous pemphigoid, pemphigoid gestationis, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, relapsing polychondritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

In certain embodiments, the autoimmune disease is an autoimmune channelopathy. In certain embodiments, the channelopathy is selected from the group consisting of autoimmune limbic encephalitis, epilepsy, neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, neuromyotonia, pediatric autoimmune neuropychiatric disorders associated with streptococcal infection (PANDAS), and Glycine receptor antibody-associated disorder. In certain embodiments, the antibody-mediated disorder is hyperglobulinemia.

In certain embodiments, the FcRn antagonist is administered to the subject simultaneously or sequentially with an additional therapeutic agent. In certain embodiments, the additional therapeutic agent is an anti-inflammatory agent. In certain embodiments, the additional therapeutic agent is a leucocyte depleting agent. In certain embodiments, the leucocyte depleting agent is a B-cell depleting agent. In certain embodiments, the B-cell depleting agent is an antibody. In certain embodiments, the B-cell depleting agent is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86. In certain embodiments, the additional therapeutic agent is rituximab, daclizumab, basiliximab, muronomab-CD3, infliximab, adalimumab, omalizumab, efalizumab, natalizumab, tocilizumab, eculizumab, golimumab, canakinumab, ustekinumab, belimumab, or a combination thereof.

In certain embodiments, the subject is a human or cynomolgus monkey.

### BRIEF DESCRIPTION OF THE DRAWINGS

FIG.1 depicts the results of experiments to determine the effect of Fc-Abdeg and HEL-Abdeg on the serum levels of a tracer antibody (FR70-hIgG1) in cynomolgous monkey.
FIG.2 depicts the results of experiments to determine the effect of Fc-Abdeg and HEL-Abdeg on total IgG serum levels in cynomolgous monkey.
FIG.3 depicts the results of experiments to determine the effect of Fc-Abdeg and HEL-Abdeg on albumin levels in cynomolgous monkey.
FIG.4 depicts the results of experiments to determine the effect of Fc-Abdeg and IVIG on the serum levels of a tracer antibody (FR70-hIgG1) in cynomolgous monkey.
FIG.5 depicts the results of ELISA assays comparing the affinity of Fc-Abdeg, Fc-Abdeg-POT and Fc-Abdeg-S239D/I332E for human CD16a.
FIG.6 depicts the results of ELISA assays comparing the affinity of Fc-Abdeg, Fc-Abdeg-POT and Fc-Abdeg-S239D/I332E for murine CD16-2.
FIG.7 depicts the results of experiments to determine the effect of Fc-Abdeg, Abdeg-POT and Fc-AbdegS239D/I332E on anti-CD20-induced ADCC-signal using the Promega's Raji-based ADCC reporter bioassay.
FIG.9 depicts the results of experiments to determine the effect of Fc-Abdeg and Abdeg- POT on anti-CD70-induced lysis of CD70+ U266 cells *in vitro.*

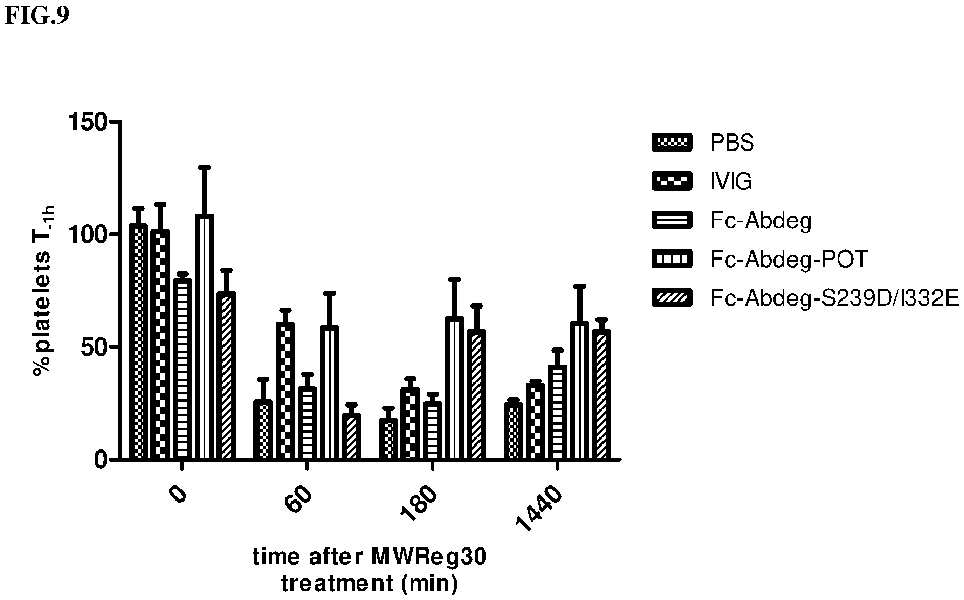

FIG.9 depicts the results of experiments to determine the effect of Fc-Abdeg, Fc-Abdeg-POT, Fc-Abdeg-S239D/I332E and IVIG on platelet levels in an acute murine model for immune thrombocytopenia.

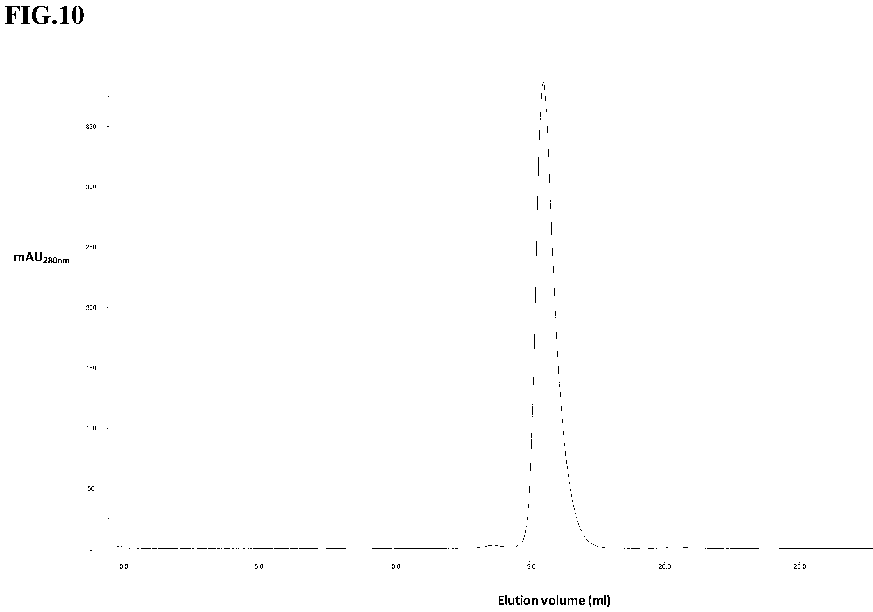

FIG.10 depicts the result of an exemplary gelfiltration purification of Fc-Abdeg.

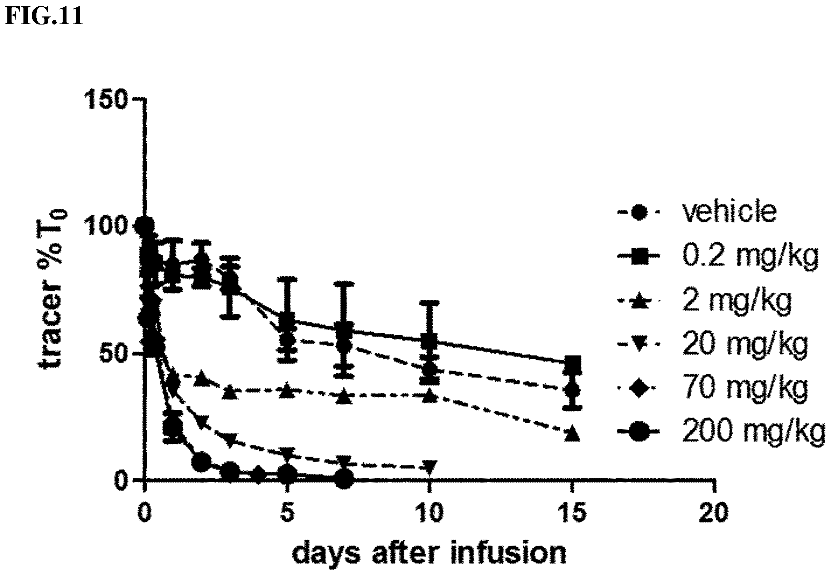

FIG.11 depicts the results of a dose-escalation study measuring the effect of various single doses of Fc-Abdeg on the serum levels of a tracer antibody (FR70-hIgG1) in cynomolgous monkey.

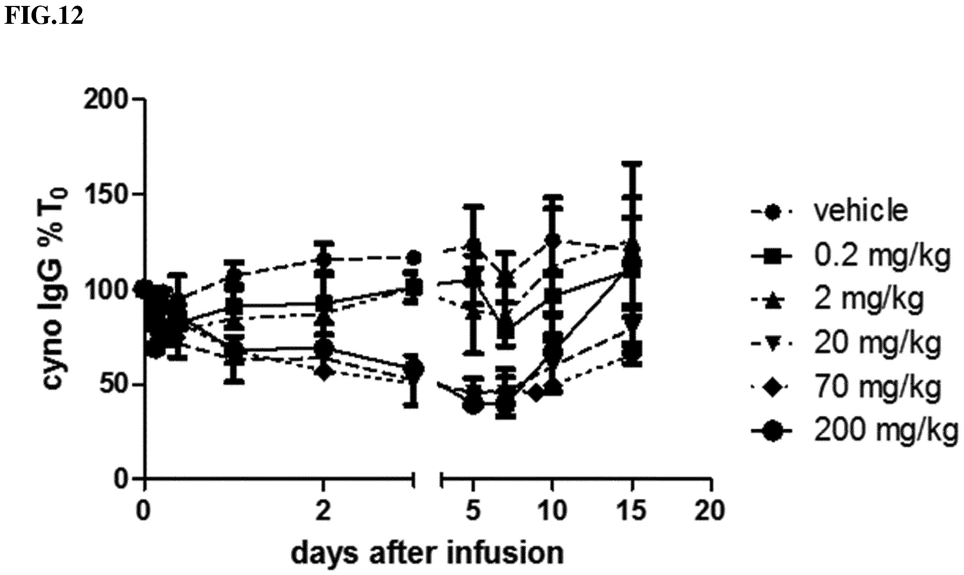

FIG.12 depicts the results of a dose-escalation study measuring the effect of various single doses of Fc-Abdeg on the serum levels of a tracer antibody (FR70-hIgG1) in cynomolgous monkey.

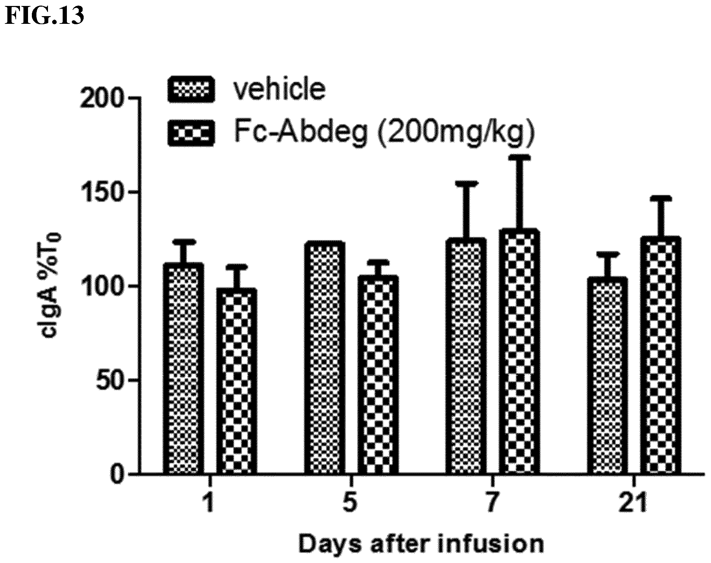

FIG.13 depicts the results of experiments to determine the effect of 200 mg/kg Fc-Abdeg on cIgA levels in cynomolgous monkey.

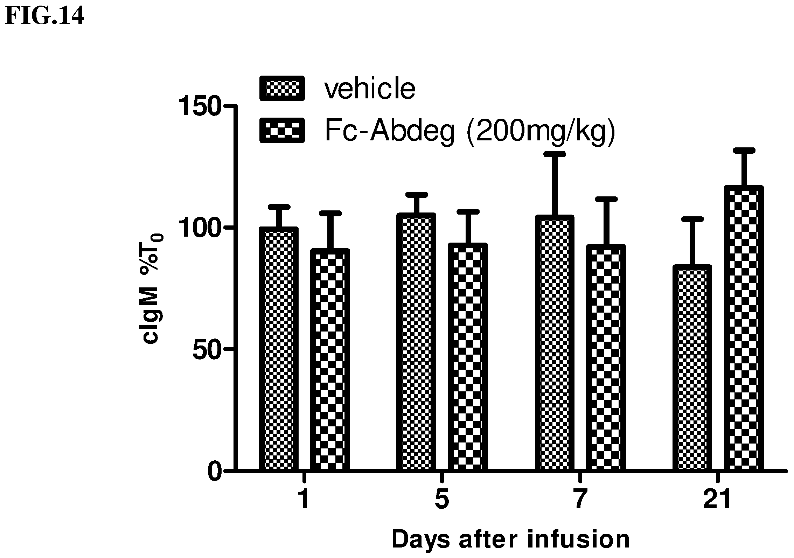

FIG.14 depicts the results of experiments to determine the effect of 200 mg/kg Fc-Abdeg on cIgM levels in cynomolgous monkey.

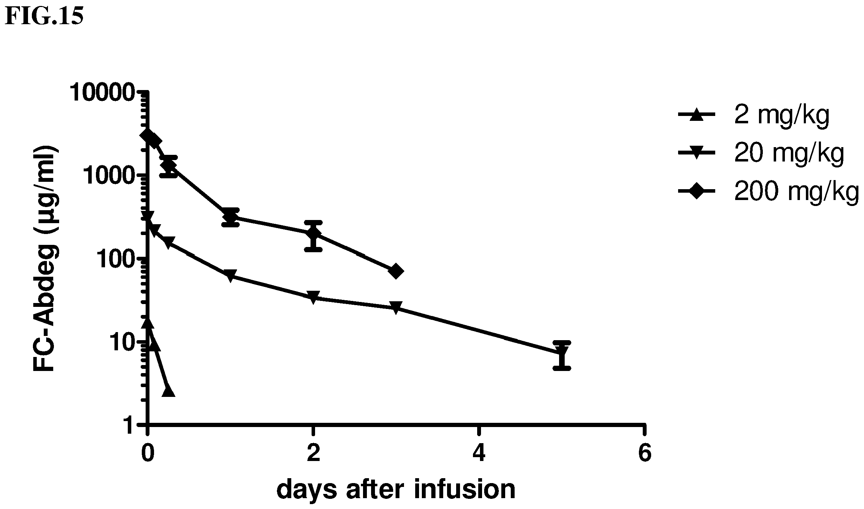

FIG.15 depicts the pharmacokinetic profile of various single doses of Fc-Abdeg in cynomolgous monkey.

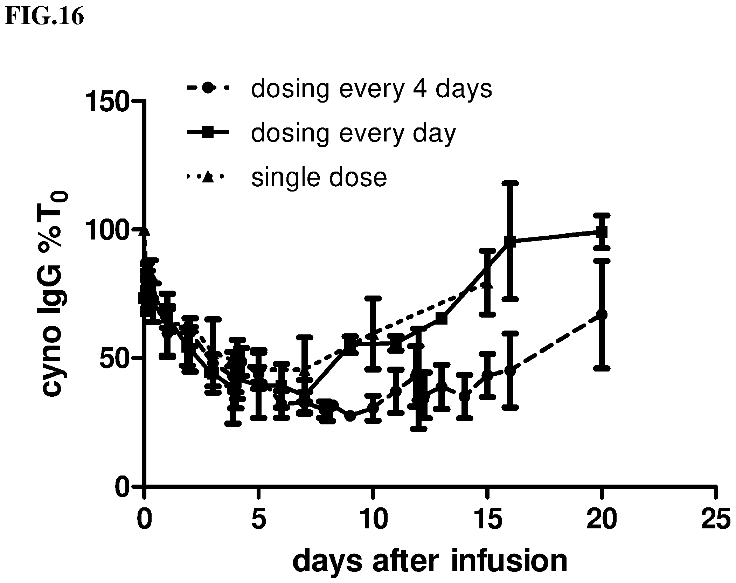

FIG.16 depicts the results of experiments to determine the effect of repetitive multiple doses of 20 mg/kg Fc-Abdeg on cIgG levels in cynomolgous monkey.

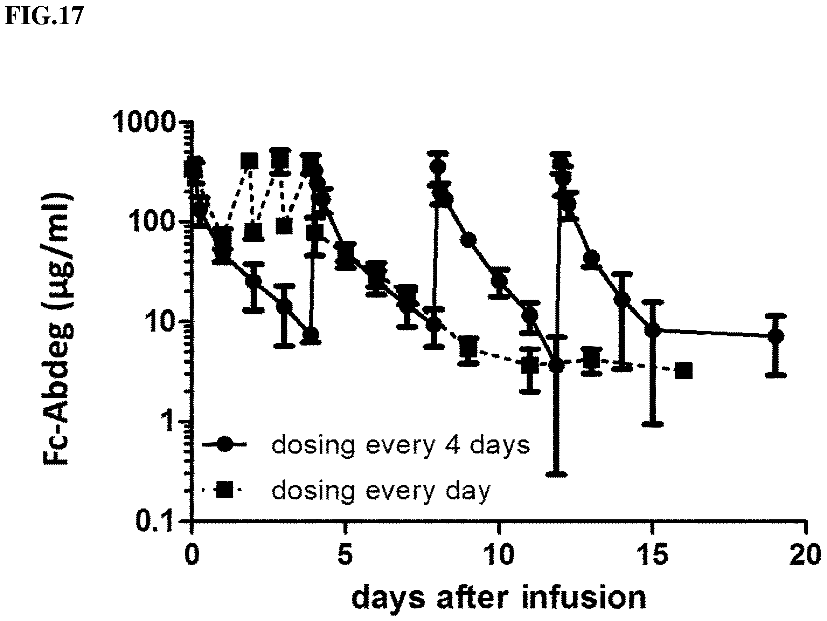

FIG.17 depicts the pharmacokinetic profile of Fc-Abdeg in cynomolgous monkey given repetitive multiple doses of 20 mg/kg Fc-Abdeg.

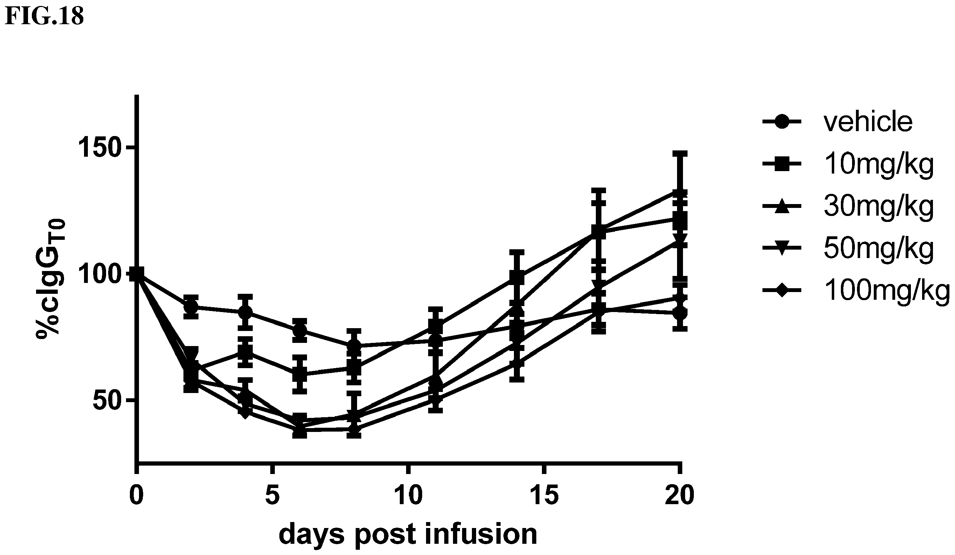

FIG.18 depicts the results of a dose-escalation study measuring the effect of various single doses of Fc-Abdeg on the serum levels of a endogenous IgG levels in cynomolgous monkey.

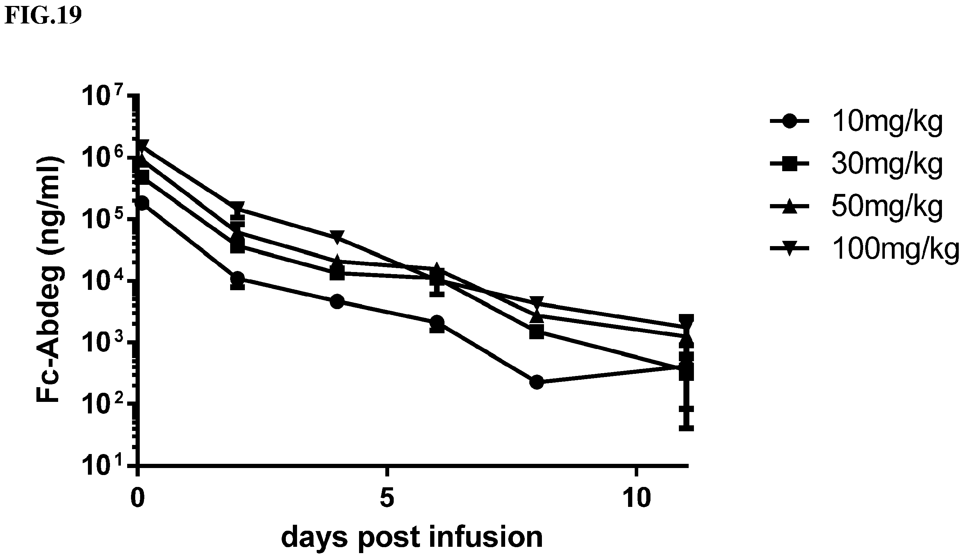

FIG. 19 depicts the pharmacokinetic profile of various single doses of Fc-Abdeg in cynomolgous monkey.

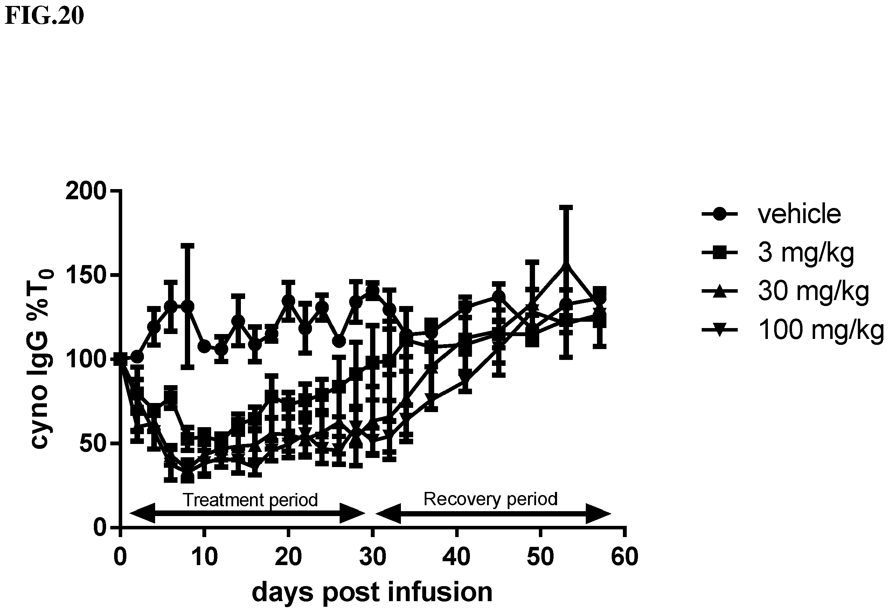

FIG.20 depicts the results of a repetitive dosing study measuring the effect of various repetitive doses of Fc-Abdeg on the serum levels of a endogenous IgG levels in cynomolgous monkey.

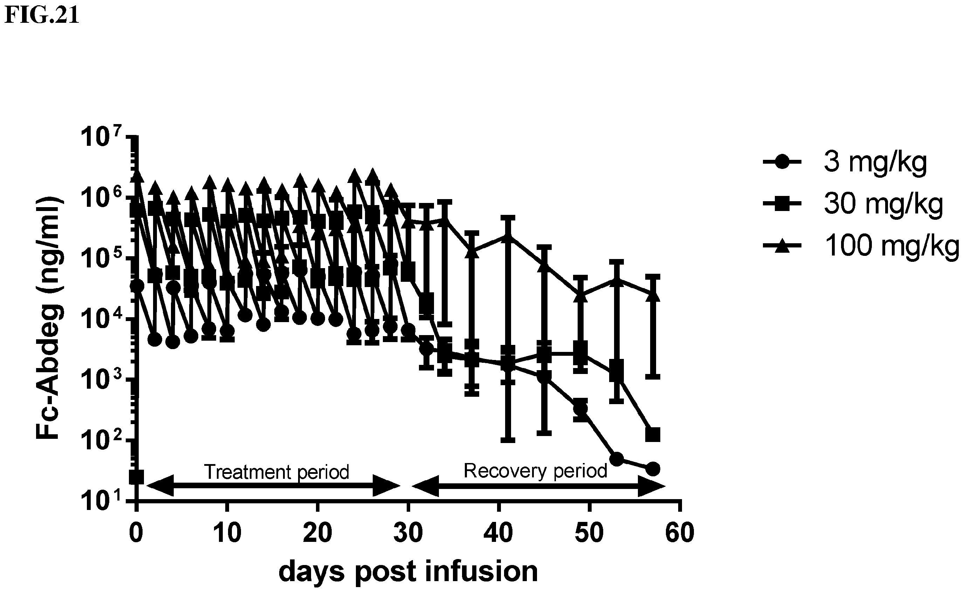

FIG.21 depicts the pharmacokinetic profile of various repetitive doses of Fc-Abdeg in cynomolgous monkey.

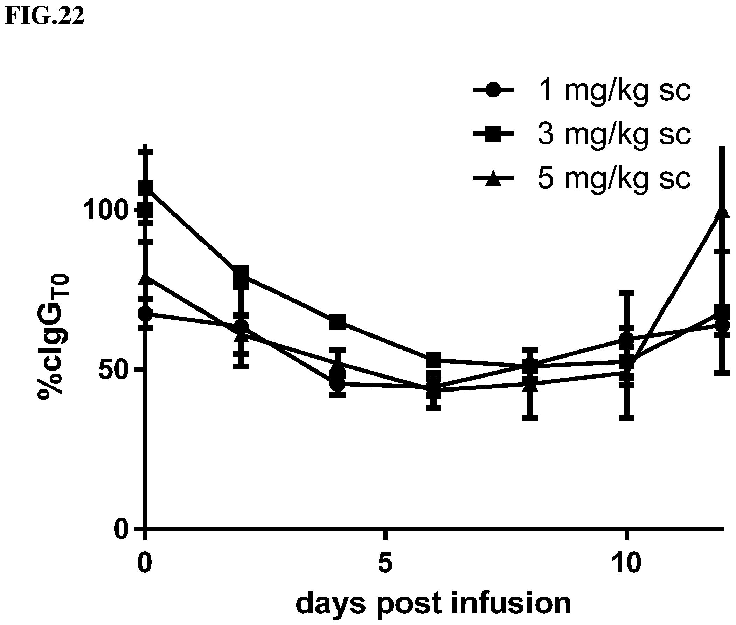

FIG.22 depicts the results of a continuous dosing study measuring the effect of various doses of Fc-Abdeg on the serum levels of a endogenous IgG levels in cynomolgous monkey.

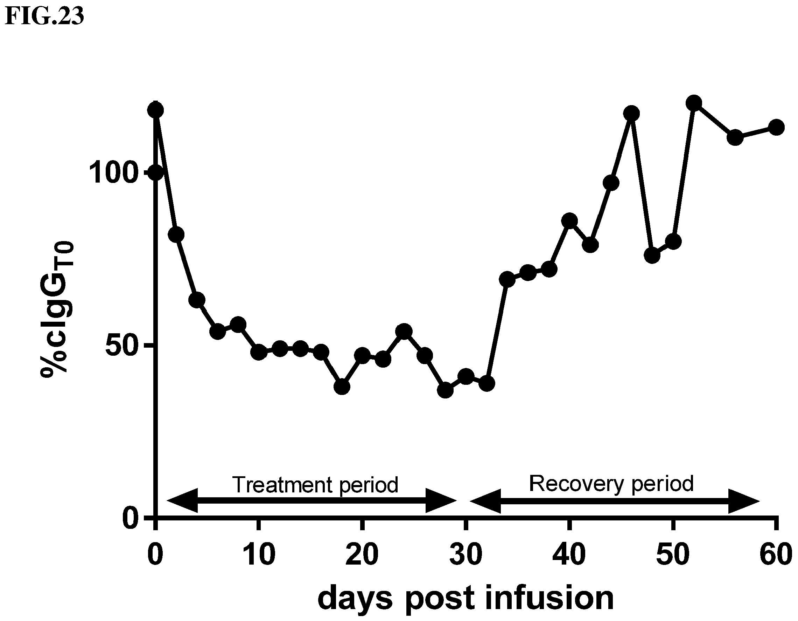

FIG.23 depicts the results of a continuous dosing study measuring the effect of an intravenous loading dose of 20 mg/kg Fc-Abdeg followed 24 hours later by daily subcutaneous administration of Fc-Abdeg at 3 mg/kg for 28 days and a subsequent treatment-free period of 32 days in a cynomolgous monkey subject.

### DETAILED DESCRIPTION

The present disclosure provides novel methods of reducing the serum levels of Fc-containing agents (e.g., antibodies and immunoadhesins) in a subject. These methods generally comprise administering to the subject an effective amount of an isolated FcRn-antagonist that binds specifically to FcRn with increased affinity and reduced pH dependence relative to the native Fc region. The disclosed methods are particularly useful for treating antibody-mediated disorders (e.g. autoimmune diseases).

### I. Definitions

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular. Generally, nomenclature used in connection with, and techniques of, cell and tissue culture, molecular biology, immunology, microbiology, genetics and protein and nucleic acid chemistry and hybridization described herein are those well known and commonly used in the art.

In order that the present invention may be more readily understood, certain terms are first defined.

As used herein the term "FcRn antagonist" refers to any agent comprising an Fc region (e.g., a variant Fc region disclosed herein) that binds specifically to FcRn through the Fc region and inhibits the binding of immunoglobulin to FcRn, with the proviso that the agent is not a full length IgG antibody.

As used herein, the term "Fc region" refers to the portion of a native immunoglobulin formed by the Fc domains of its two heavy chains. A native Fc region is homodimeric.

As used herein, the term "variant Fc region" refers to an Fc region with one or more alteration relative to a native Fc region. Alteration can include amino acid substitutions, additions and/or deletions, linkage of additional moieties, and/or alteration the native glycans. The term encompasses heterodimeric Fc regions where each of the constituent Fc domains is different. Examples of such heterodimeric Fc regions include, without limitation, Fc regions made using the "knobs and holes" technology as described in, for example, US 8216805, which is incorporated by reference herein in its entirety. The term also encompasses single chain Fc regions where the constituent Fc domains are linked together by a linker moiety, as described in, for example, US20090252729A1 and US20110081345A1, which are each incorporated by reference herein in their entirety.

As used herein, the term "Fc domain" refers to the portion of a single immunoglobulin heavy chain beginning in the hinge region just upstream of the papain cleavage site and ending at the C-terminus of the antibody. Accordingly, a complete Fc domain comprises at least a portion of a hinge (e.g., upper, middle, and/or lower hinge region) domain, a CH2 domain, and a CH3 domain.

As used herein the term "FcRn binding fragment" refers to a portion of an Fc region that is sufficient to confer FcRn binding.

As used herein, the term "EU position" refers to the amino acid position in the EU numbering convention for the Fc region described in Edelman, G.M. et al., Proc. Natl. Acad. USA, 63, 78-85 (1969) and Kabat et al, in "Sequences of Proteins of Immunological Interest", U.S. Dept. Health and Human Services, 5th edition, 1991.

As used herein, the term "CH1 domain" refers to the first (most amino terminal) constant region domain of an immunoglobulin heavy chain that extends from about EU positions 118-215. The CH1 domain is adjacent to the VH domain and amino terminal to the hinge region of an immunoglobulin heavy chain molecule, and does not form a part of the Fc region of an immunoglobulin heavy chain.

As used herein, the term "hinge region" refers to the portion of a heavy chain molecule that joins the CH1 domain to the CH2 domain. This hinge region comprises approximately 25 residues and is flexible, thus allowing the two N-terminal antigen binding regions to move independently. Hinge regions can be subdivided into three distinct domains: upper, middle, and lower hinge domains (Roux et al. J. Immunol. 161: 4083 (1998)). The FcRn antagonists of the instant disclosure can include all or a portion of a hinge region.

As used herein, the term "CH2 domain" refers to the portion of a heavy chain immunoglobulin molecule that extends from about EU positions 231-340.

As used herein, the term "CH3 domain" includes the portion of a heavy chain immunoglobulin molecule that extends approximately 110 residues from N-terminus of the CH2 domain, e.g., from about position 341-446 (EU numbering system).

As used herein, the term "FcRn" refers to a neonatal Fc receptor. Exemplary FcRn molecules include human FcRn encoded by the FCGRT gene as set forth in RefSeq NM_004107.

As used herein, the term "CD16" refers to FcyRIII Fc receptors that are required for Antibody-Dependent Cell-mediated Cytotoxicity (ADCC). Exemplary CD16 molecules include human CD16a as set forth in RefSeq NM_000569.

As used herein, the term "free cysteine" refers to native or engineered cysteine amino acid residue that exists in a substantially reduced form in a mature FcRn antagonist.

As used herein, the term "antibody" refers to immunoglobulin molecules comprising four polypeptide chains, two heavy (H) chains and two light (L) chains interconnected by disulfide bonds, as well as multimers thereof (e.g., IgM). Each heavy chain comprises a heavy chain variable region (abbreviated VH) and a heavy chain constant region. The heavy chain constant region comprises three domains, CH1, CH2 and CH3. Each light chain comprises a light chain variable region (abbreviated VL) and a light chain constant region. The light chain constant region comprises one domain (CL). The VH and VL regions can be further subdivided into regions of hypervariability, termed complementarity determining regions (CDRs), interspersed with regions that are more conserved, termed framework regions (FR).

As used herein the term "N-linked glycan" refers to the N-linked glycan attached to the nitrogen (N) in the side chain of asparagine in the sequon (i.e., Asn-X-Ser or Asn-X-Thr sequence, where X is any amino acid except proline) present in the CH2 domain of an Fc region. Such N-Glycans are fully described in, for example, Drickamer K, Taylor ME (2006). Introduction to Glycobiology, 2nd ed., which is incorporated herein by reference in its entirety.

As used herein the term "afucosylated" refers to an N-linked glycan which lacks a core fucose molecule as described in US8067232, the contents of which is incorporated by reference herein in its entirety.

As used herein the term "bisecting GlcNac" refers to an N-linked glycan having an N-acetylglucosamine (GlcNAc) molecule linked to a core mannose molecule, as described in US8021856, the contents of which is incorporated by reference herein in its entirety.

As used herein, the term "antibody-mediated disorder" refers to any disease or disorder caused or exacerbated by the presence of an antibody in a subject.

As used herein, the term "Fc-containing agent" is any molecule that comprises an Fc region.

As used herein, the term "leucocyte depleting agent" refers to an agent that reduces the number of leucocytes in a subject upon administration.

As used herein, the term "B-cell depleting agent" refers to an agent that reduces the number of B-cells in a subject upon administration.

As used herein, the term "T-cell depleting agent" refers to an agent that reduces the number of T-cells in a subject upon administration.

As used herein, the term "autoimmune channelopathy" refers to a diseases caused by autoantibodies against an ion channel subunit or a molecule that regulates the channel.

As used herein, the term "treat," "treating," and "treatment" refer to therapeutic or preventative measures described herein. The methods of "treatment" employ administration to a subject, an antibody or antigen binding fragment thereof of the present invention, for example, a subject having an IL-6-associated disease or disorder (e.g. inflammation and cancer) or predisposed to having such a disease or disorder, in order to prevent, cure, delay, reduce the severity of, or ameliorate one or more symptoms of the disease or disorder or recurring disease or disorder, or in order to prolong the survival of a subject beyond that expected in the absence of such treatment. As used herein, the term "subject" includes any human or non-human animal.

As used herein, the term "immunoadhesin" refers to an antibody-like molecule, which comprises a functional domain of a binding protein (e.g., a receptor, ligand, or cell-adhesion molecule) with an Fc region.

### II. Methods of Reducing Serum Levels of Fc-Containing Agents

In one aspect, the instant disclosure provides methods of reducing the serum levels of Fc-containing agents (e.g., antibodies and immunoadhesins) in a subject, the methods comprising administering to the subject an effective amount of an isolated FcRn-antagonist that binds specifically to FcRn with increased affinity and reduced pH dependence relative to the native Fc region (e.g., FcRn-antagonists disclosed herein).

As shown herein, administration of an FcRn-antagonist to the subject in a dose of between about 0.2 and about 200 mg/kg is unexpectedly efficacious. Accordingly, in certain embodiments, the FcRn-antagonist is administered to the subject in a dose of between about 0.2 and about 200 mg/kg (e.g., between 0.2 and 200 mg/kg). In certain embodiments, the FcRn-antagonist is administered to the subject in a dose of about 0.2, 2, 20, 70, or 200 mg/kg (e.g., 0.2, 2, 20, 70, or 200 mg/kg). In certain embodiments, the FcRn-antagonist is administered to the subject in a dose of about 20 mg/kg (e.g., 20 mg/kg).

As shown herein, a multiple, repeated dosing regime is unexpectedly superior to a single dose. Accordingly, in certain embodiments, the FcRn-antagonist is administered to the subject at least twice in 20 days. In certain embodiments, the FcRn-antagonist is administered to the subject at a frequency of once every 1, 2, 3, 4, 5, 6, 7, 8, 9, or 10 days. In certain embodiments, the FcRn-antagonist is administered to the subject 2, 3, 4, 5, 6, 7, 8, 9, 10, 11, 12, 13, 14, 15, 16, 17, 18, 19, or 20 times in 20 days. In certain embodiments, the FcRn-antagonist is administered to the subject at a frequency of once every 4 days. In certain embodiments, the FcRn-antagonist is administered to the subject every 4 days for 13 days (i.e., on days 1, 5, 9, and 13). In certain embodiments, 20 mg/kg of the FcRn-antagonist is administered to the subject every 4 days for 13 days (i.e., on days 1, 5, 9, and 13).

The FcRn-antagonist can be administered by any means to the subject. Methods of administration include, but are not limited to, intradermal, intramuscular, intraperitoneal, intravenous, subcutaneous, intranasal, epidural, and oral routes. The composition may be administered, for example by infusion or bolus injection. In certain embodiments, the FcRn-antagonist is administered by intravenous infusion.

The methods disclosed herein can reduce the serum levels of any Fc-containing agent. In certain embodiments, the Fc-containing agent is an antibody or immunoadhesin. In certain embodiments, the Fc-containing agent is a therapeutic or diagnostic agent. In certain embodiments, the Fc-containing agent is an imaging agent. In certain embodiments the Fc-containing agent is an antibody-drug conjugate. In certain embodiments, the Fc-containing agent is a pathogenic antibody, e.g., an autoantibody. In certain embodiments, the subject has an antibody-mediated disorder a disease or disorder. In certain embodiments, antibody-mediated disorder a disease or disorder is associated with an autoantibody.

The reduction of serum levels of Fc-containing agents (e.g., antibodies and immunoadhesins) is particularly applicable to the treatment of antibody-mediated disorders (e.g. autoimmune diseases). Accordingly, in one aspect the instant disclosure provides methods of treating a subject having an antibody-mediated disorder (e.g. an autoimmune disease), the method comprising administering to the subject an effective amount of an FcRn antagonist composition disclosed herein.

Any antibody-mediated disorder can be treated using the methods disclosed herein. In certain embodiments, the antibody-mediated disorder is one that is amenable to treatment by IVIG. In certain embodiments, the antibody-mediated disorder is an autoimmune disease. Non-limiting autoimmune diseases include allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, discoid lupus, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lupus erthematosis, Meniere's disease, mixed connective tissue disease, multiple sclerosis, type 1 diabetes mellitus, Multifocal motor neuropathy (MMN), myasthenia gravis, paraneoplastic bullous pemphigoid, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

In certain embodiments, the autoimmune disease is an autoimmune channelopathy. Non-limiting channelopathies include neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, and Glycine receptor antibody-associated disorder.

The methods of the instant disclosure are particularly suited to treating antibody-mediated disorders characterized by an over production of serum immunoglobulin. Accordingly, in certain embodiments, the FcRn antagonist compositions are used to treat hypergammaglobulinemia.

The methods of the instant disclosure can also be used in combination with one or more additional therapeutic agents. In certain embodiments, the additional therapeutic agent is an anti-inflammatory agent. Any inflammatory agent can be used in combination with the compositions disclosed herein. In certain embodiments, the therapeutic agent is rituximab, daclizumab, basiliximab, muronomab-cd3, infliximab, adalimumab, omalizumab, efalizumab, natalizumab, tocilizumab, eculizumab, golimumab, canakinumab, ustekinumab, or belimumab. In certain embodiments, the additional therapeutic agent is leucocyte depleting agent (e.g., B-cell or T-cell depleting agent). Any leucocyte depleting agent can be used in combination with the FcRn antagonist compositions disclosed herein. In certain embodiments, the leucocyte depleting agent is a B-cell depleting agent. In certain embodiments, the leucocyte depleting agent is an antibody against a cell surface marker. Suitable cell surface markers include, without limitation, CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86. The FcRn antagonist and the additional therapeutic agent(s) can be administered to the subject simultaneously or sequentially, via the same or different route(s) of administration.

The methods of the instant disclosure are also well suited to rapidly reducing the serum levels of an Fc-containing agent in subject. Such rapid clearance is advantageous in cases where the Fc-containing agent is toxic (e.g., an antibody-drug conjugate or an agent that is immunogenic) because it reduces the exposure of the subject to the drug. Rapid clearance is also advantageous in cases where the Fc-containing agent is an imaging agent that requires a low serum level of the agent to facilitate imaging. Accordingly, in certain embodiments, the FcRn antagonist compositions are used to reduce the serum levels of an Fc-containing agent (e.g., an imaging agent) in subject that has been administered the Fc-containing agent. The serum levels of any Fc-containing agent (e.g., therapeutic or diagnostic agent) can be reduced using the FcRn antagonist compositions disclosed herein. Non limiting examples of Fc-containing agents include imaging agents (e.g., labeled antibodies), antibody drug conjugates, or immunogenic agents (e.g., non-human antibodies or immunoadhesins). The FcRn antagonist can be administered simultaneously with the Fc-containing agent or sequentially (e.g., before or after the Fc-containing agent).

Furthermore, in diseases or conditions requiring administration of a therapeutic agent, the subject will often develop antibodies (e.g., anti-drug antibodies) against the therapeutic agent, which, in turn, prevent the therapeutic agent from being available for its intended therapeutic purpose or cause an adverse reaction in the subject. Accordingly, the methods disclosed herein can also be used to remove antibodies (e.g., anti-drug antibodies) against the therapeutic agent that develop in a subject.

The methods disclosed herein can also be used in combination with a therapeutic protein to enhance the benefit of the therapeutic protein by reducing the levels of IgG; wherein, IgG antibodies are responsible for the decreased bioavailability of a therapeutic protein. In certain embodiments the instant disclosure provides a method of treating a subject having a disorder resulting from an immune response to a clotting factor, the method comprising administering to a subject a therapeutically effective amount of an FcRn antagonist compositions disclosed herein. Suitable clotting factors include, without limitation, fibrinogen, prothrombin, factor V, factor VII, factor VIII, factor IX, factor X, factor XI, factor XII, factor XIII, or von Willebrand's factor. This method may be used to regulate or treat, or prevent an immune response to a clotting factor in a patient suffering, e.g., from hemophilia A or hemophilia B. In certain embodiments, the method may be used to regulate or treat an immune response to, e.g., therapeutic erythropoietin in a patient suffering from pure red cell aplasia (PRCA).

FcRn is responsible for transporting maternal antibodies across the placenta to the fetus in a pregnant woman. Accordingly, if a pregnant female is administered an Fc-containing agent (e.g., a therapeutic antibody), the agent may come in contact with the fetus as a result of the FcRn-mediated transport across the placenta. To avoid any potential deleterious effect of the Fc-containing agent on fetal development, it would be advantageous to block FcRn function. Accordingly, the instant disclosure provides a method of preventing placental transfer of an Fc-containing agent (e.g., a therapeutic antibody) to the fetus in a pregnant woman, the method comprising administering to the woman an FcRn antagonist compositions disclosed herein, either simultaneously or sequentially (prior or post) with the Fc-containing agent.

The methods disclosed herein can also be used to treat inflammatory disorders including, but not limited to, asthma, ulcerative colitis and inflammatory bowel syndrome allergy, including allergic rhinitis/sinusitis, skin allergies (urticaria/hives, angioedema, atopic dermatitis), food allergies, drug allergies, insect allergies, mastocytosis, arthritis, including osteoarthritis, rheumatoid arthritis, and spondyloarthropathies.

Successful implementation of gene therapy for the treatment of a disease or condition may be hampered by the development of antibodies specific to the therapeutic protein encoded by the transgene as well as possibly to the vector used to deliver the transgene. Accordingly, the FcRn antagonist compositions disclosed herein can be administered in combination with gene therapy to enhance the benefit of the encoded therapeutic protein by reducing the levels of IgG. These methods are particularly useful in situations where IgG antibodies are responsible for the decreased bioavailability of a gene therapy vector or the encoded therapeutic protein. The gene therapy vector may be, e.g., a viral vector such as adenovirus and adeno-associated virus. Diseases that can be treated using gene therapy include, but are not limited to, cystic fibrosis, hemophilia, PRCA, muscular dystrophy, or lysosomal storage diseases, such as, e.g., Gaucher's disease and Fabry's disease.

Any subject can be treated using the methods disclosed herein. In certain embodiments, the subject is a human or a cynomolgus monkey.

### III. FcRn Antagonists

The methods disclosed herein generally comprise administering to a subject an effective amount of an isolated FcRn-antagonist, wherein the FcRn-antagonist binds specifically to FcRn with increased affinity and reduced pH dependence relative to the native Fc region. In general, these FcRn-antagonists comprise a variant Fc region, or FcRn-binding fragment thereof, that binds specifically to FcRn with increased affinity and reduced pH dependence relative to a native Fc region. The FcRn antagonists inhibit the binding of Fc-containing agents (e.g., antibodies and immunoadhesins) to FcRn *in vivo,* which results in an increased rate of degradation of the Fc-containing agents and, concommitantly, a reduced serum level of these agents.

As shown herein, an isolated variant Fc region (e.g., a variant Fc region comprising the amino acids Y, T, E, K, F, and Y at EU positions 252, 254, 256, 433, 434, and 436 respectively) is a more efficacious FcRn antagonist *in vivo* than a full-length antibody comprising the same variant Fc region. Accordingly, in certain embodiments, the FcRn antagonist compositions are not full-length antibodies. In certain embodiments, the FcRn antagonist compositions do not comprise an antibody variable domain. In certain embodiments, the FcRn antagonist compositions do not comprise an antibody variable domain or a CH1 domain. However, in certain embodiments, the FcRn antagonist compositions may comprise a variant Fc region linked to one or more additional binding domains or moieties, including antibody variable domains.

Any Fc region can be altered to produce a variant Fc region for use in the FcRn antagonist compositions disclosed herein. In general, an Fc region, or FcRn-binding fragment thereof, is from a human immunoglobulin. It is understood, however, that the Fc region may be derived from an immunoglobulin of any other mammalian species, including for example, a Camelid species, a rodent (e.g. a mouse, rat, rabbit, guinea pig) or non-human primate (e.g. chimpanzee, macaque) species. Moreover, the Fc region or portion thereof may be derived from any immunoglobulin class, including IgM, IgG, IgD, IgA and IgE, and any immunoglobulin isotype, including IgGl, IgG2, IgG3 and IgG4. In certain embodiments, the Fc region is an IgG Fc region (e.g., a human IgG region). In certain embodiments, the Fc region is an IgG1 Fc region (e.g., a human IgG1 region). In certain embodiments, the Fc region is a chimeric Fc region comprising portions of several different Fc regions. Suitable examples of chimeric Fc regions are set forth in US20110243966A1, which is herein incorporated by reference in its entirety. A variety of Fc region gene sequences (e.g. human constant region gene sequences) are available in the form of publicly accessible deposits. It will be appreciated that the scope of this invention encompasses alleles, variants and mutations of Fc regions.

An Fc region can be further truncated or internally deleted to produce a minimal FcRn-binding fragment thereof. The ability of an Fc-region fragment to bind to FcRn can be determined using any art recognized binding assay e.g., ELISA.

To enhance the manufacturability of the FcRn antagonists disclosed herein, it is preferable that the constituent Fc regions do not do comprise any non-disulphide bonded cysteine residues. Accordingly, in certain embodiments the Fc regions do not comprise a free cysteine residue.

Any Fc variant, or FcRn-binding fragment thereof, that binds specifically to FcRn with increased affinity and reduced pH dependence relative to the native Fc region can be used in the FcRn antagonist compositions disclosed herein. In certain embodiments, the variant Fc region comprises amino acid alterations, substitutions, insertions and/or deletions that confer the desired characteristics. In certain embodiments, the variant Fc region or fragment comprises the amino acids Y, T, E, K, F, and Y at EU positions 252, 254, 256, 433, 434, and 436 respectively. Non-limiting examples of amino acid sequences that can be used in variant Fc regions are set forth in Table 1, herein. In certain embodiments, the amino acid sequence of the Fc domains of the variant Fc region comprises the amino acid sequence set forth in SEQ ID NO:1. In certain embodiments, the amino acid sequence of the Fc domains of the variant Fc region consists of the amino acid sequence set forth in SEQ ID NO:1, 2, or 3. In certain embodiments an FcRn-antagonist consists of a variant Fc region, wherein the amino acid sequence of the Fc domains of the variant Fc region consists of the amino acid sequence set forth in SEQ ID NO:1, 2, or 3.

**Table 1. Amino acid sequences of non-limiting examples of variant Fc regions**

| **SEQ ID NO** | **Amino Acid Sequence** |
|---|---|
| 1 | CPPCPAPELLGGPSVFLFPPKPKDTLYITREPEVTCVVVDVSHEDPEVKFNWYVD GVEVHNAKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNGQPENN YKTTPPVLDSDGSFFLYSKLTVDKSRWQQGNVFSCSVMHEALKFHYTQKSLSLSP G |
| 2 | DKTHTCPPCPAPELLGGPSVFLFPPKPKDTLYITREPEVTCVVVDVSHEDPEVKF NWYVDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALKFHYTQKS LSLSPGK |
| 3 | DKTHTCPPCPAPELLGGPSVFLFPPKPKDTLYITREPEVTCVVVDVSHEDPEVKF NWYVDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALKFHYTQKS LSLSPG |
| Amino acids at EU positions 252, 254, 256, 433, and 434 are underlined | |

In certain embodiments, the variant Fc region has altered (e.g., increased or decreased) binding affinity for an additional Fc receptor. The variant Fc region can have altered (e.g., increased or decreased) binding affinity for one or more of Fcγ receptors e.g., FcyRI (CD64), FcyRIIA (CD32), FcγRIIB (CD32), FcyRIIIA (CD16a), and FcγRIIIB (CD16b). Any art recognized means of altering the affinity for an additional Fc receptor can be employed. In certain embodiments, the amino acid sequence of the variant Fc region is altered.

In certain embodiments, the variant Fc region comprises a non-naturally occurring amino acid residue at one or more positions selected from the group consisting of 234, 235, 236, 239, 240, 241, 243, 244, 245, 247, 252, 254, 256, 262, 263, 264, 265, 266, 267, 269, 296, 297, 298, 299, 313, 325, 326, 327, 328, 329, 330, 332, 333, and 334 as numbered by the EU index as set forth in Kabat. Optionally, the Fc region may comprise a non-naturally occurring amino acid residue at additional and/or alternative positions known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752 and WO 05/040217, the contents of which are incorporated by reference herein in their entirety).

In certain embodiments, the variant Fc region comprises at least one non-naturally occurring amino acid residue selected from the group consisting of 234D, 234E, 234N, 234Q, 234T, 234H, 234Y, 2341, 234V, 234F, 235A, 235D, 235R, 235W, 235P, 235S, 235N, 235Q, 235T, 235H, 235Y, 2351, 235V, 235F, 236E, 239D, 239E, 239N, 239Q, 239F, 239T, 239H, 239Y, 2401, 240A, 240T, 240M, 241W, 241 L, 241Y, 241E, 241R. 243W, 243L 243Y, 243R, 243Q, 244H, 245A, 247V, 247G, 252Y, 254T, 256E, 2621, 262A, 262T, 262E, 2631, 263A, 263T, 263M, 264L, 2641, 264W, 264T, 264R, 264F, 264M, 264Y, 264E, 265G, 265N, 265Q, 265Y, 265F, 265V, 2651, 265L, 265H, 265T, 2661, 266A, 266T, 266M, 267Q, 267L, 269H, 269Y, 269F, 269R, 296E, 296Q, 296D, 296N, 296S, 296T, 296L, 2961, 296H, 269G, 297S, 297D, 297E, 298H, 2981, 298T, 298F, 2991, 299L, 299A, 299S, 299V, 299H, 299F, 299E, 313F, 325Q, 325L, 3251, 325D, 325E, 325A, 325T, 325V, 325H, 327G, 327W, 327N, 327L, 328S, 328M, 328D, 328E, 328N, 328Q, 328F, 3281, 328V, 328T, 328H, 328A, 329F, 329H, 329Q, 330K, 330G, 330T, 330C, 330L, 330Y, 330V, 3301, 330F, 330R, 330H, 332D, 332S, 332W, 332F, 332E, 332N, 332Q, 332T, 332H, 332Y, and 332A as numbered by the EU index as set forth in Kabat. Optionally, the Fc region may comprise additional and/or alternative non-naturally occurring amino acid residues known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752 and WO 05/040217, the contents of which are incorporated by reference herein in their entirety).

Other known Fc variants that may be used in the FcRn antagonists disclosed herein include without limitations those disclosed in Ghetie et al., 1997, Nat. Biotech. 15:637-40; Duncan et al, 1988, Nature 332:563-564; Lund et al., 1991, J. Immunol., 147:2657-2662; Lund et al, 1992, Mol. Immunol., 29:53-59; Alegre et al, 1994, Transplantation 57:1537-1543; Hutchins et al., 1995, Proc Natl. Acad Sci USA, 92:11980-11984; Jefferis et al, 1995, Immunol Lett., 44:111-117; Lund et al., 1995, Faseb J., 9:115-119; Jefferis et al, 1996, Immunol Lett., 54:101-104; Lund et al, 1996, J. Immunol., 157:4963-4969; Armour et al., 1999, Eur J Immunol 29:2613-2624; Idusogie et al, 2000, J. Immunol., 164:4178-4184; Reddy et al, 2000, J. Immunol., 164:1925-1933; Xu et al., 2000, Cell Immunol., 200:16-26; Idusogie et al, 2001, J. Immunol., 166:2571-2575; Shields et al., 2001, J Biol. Chem., 276:6591-6604; Jefferis et al, 2002, Immunol Lett., 82:57-65; Presta et al., 2002, Biochem Soc Trans., 30:487-490); U.S. Pat. Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351, the contents of which are incorporated by reference herein in their entirety.

In certain embodiments, the variant Fc region is a heterodimer, where the constituent Fc domains are different from each other. Methods of producing Fc heterodimers are known in the art (see e.g., US 8216805, which is incorporated by reference herein in its entirety). In certain embodiments, the variant Fc region is a single chain Fc region, where the constituent Fc domains are linked together by a linker moiety. Methods of producing single chain Fc regions are known in the art (see e.g., US20090252729A1 and US20110081345A1, which are each incorporated by reference herein in their entirety).

It is believed that pathogenic IgG antibodies observed in autoimmune diseases are either the pathogenic triggers for these diseases or contribute to disease progression and mediate disease through the inappropriate activation of cellular Fc receptors. Aggregated autoantibodies and/or autoantibodies complexed with self antigens (immune complexes) bind to activating Fc receptors, causing numerous autoimmune diseases (which occur in part because of immunologically mediated inflammation against self tissues) (see e.g., Clarkson et al., NEJM 314(9), 1236-1239 (2013)); US20040010124A1; US20040047862A1; and US2004/0265321A1, which are each incorporated by reference herein in their entirety). Accordingly, to treat antibody-mediated disorders (e.g. autoimmune diseases), it would be advantageous to both remove the deleterious autoantibodies and to block the interaction of the immune complexes of these antibodies with activating Fc receptors (e.g., Fcγ receptors, such as CD16a).

Accordingly, in certain embodiments, the variant Fc region of the FcRn antagonist exhibits increased binding to CD16a (e.g., human CD16a). This is particularly advantageous in that it allows the FcRn antagonist to additionally antagonize the immune complex-induced inflammatory response of autoantibodies being targeted for removal by FcRn inhibition. Any art recognized means of increasing affinity for CD16a (e.g., human CD16a) can be employed. In certain embodiments, the FcRn-antagonist comprises a variant Fc-region comprising an N-linked glycan (e.g., at EU position 297). In this case it is possible to increase the binding affinity of the FcRn-antagonist for CD16a by altering the glycan structure. Alterations of the N-linked glycan of Fc regions are well known in the art. For example, afucosylated N-linked glycans or N-glycans having a bisecting GlcNac structure have been shown to exhibit increased affinity for CD16a. Accordingly, in certain embodiments, the N-linked glycan is afucosylated. Afucosylation can be achieved using any art recognized means. For example, an FcRn-antagonist can be expressed in cells lacking fucosyl transferase, such that fucose is not added to the N-linked glycan at EU position 297 of the variant Fc region (see e.g., US 8,067,232, the contents of which is incorporated by reference herein in its entirety). In certain embodiments, the N-linked glycan has a bisecting GlcNac structure. The bisecting GlcNac structure can be achieved using any art recognized means. For example, an FcRn-antagonist can be expressed in cells expressing betal-4-N-acetylglucosaminyltransferase III (GnTIII) , such that bisecting GlcNac is added to the N-linked glycan at EU position 297 of the variant Fc region (see e.g., US 8021856, the contents of which is incorporated by reference herein in its entirety). Additionally or alternatively, alterations of the N-linked glycan structure can also be achieved by enzymatic means *in vitro.*

In certain embodiments, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region, or FcRn-binding fragment thereof, comprising an afucosylated N-linked glycan at EU position 297.

In certain embodiments, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region or FcRn-binding fragment thereof, comprising an N-linked glycan having a bisecting GlcNac at EU position 297.

In certain embodiments, the variant Fc region does not comprise an N-linked glycan. This can be achieved using any art recognized methods. For example, the Fc variant can be expressed in a cell that is incapable of N-linked glycosylation. Additionally or alternatively, the amino acid sequence of the Fc variant can be altered to prevent or inhibit N-linked glycosylation (e.g., by mutation of the NXT sequon). Alternatively, the Fc variant can be synthesized in an acellular system (e.g., chemically synthesized).

In certain embodiments, FcRn-antagonist molecules may be modified, e.g., by the covalent attachment of a molecule (e.g., a binding or imaging moiety) to the FcRn-antagonist such that covalent attachment does not prevent the FcRn-antagonist from specifically binding to FcRn. For example, but not by way of limitation, the FcRn-antagonist may be modified by glycosylation, acetylation, pegylation, phosphorylation, amidation, derivatization by known protecting blocking groups, proteolytic cleavage, linkage to a cellular ligand or other protein, etc.

In certain embodiments, the FcRn antagonist comprises a variant Fc region linked to a half-life extender. As used herein, the term "half-life extender" refers to any molecule that, when linked to an FcRn antagonist disclosed herein, increases the half-life of an FcRn antagonist. Any half-life extender may be linked (either covalently or non-covalently) to the FcRn antagonist. In certain embodiments, the half-life extender is polyethylene glycol or human serum albumin. In certain embodiments, the FcRn antagonist is linked to a binding molecule that specifically binds to a half-life extender present in a subject, such as a blood-carried molecule or cell, such as serum albumin (e.g., human serum albumin), IgG, erythrocytes, etc.

### IV. Pharmaceutical Compositions

In certain embodiments, the methods employ pharmaceutical compositions comprising an FcRn antagonist or FcRn antagonist composition disclosed herein and a pharmaceutically acceptable carrier or excipient. Examples of suitable pharmaceutical carriers are described in Remington's Pharmaceutical Sciences by E. W. Martin. Examples of excipients can include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition can also contain pH buffering reagents, and wetting or emulsifying agents.

The pharmaceutical composition can be formulated for parenteral administration (e.g., intravenous or intramuscular) by bolus injection. Formulations for injection can be presented in unit dosage form, e.g., in ampoules or in multidose containers with an added preservative. The compositions can take such forms as suspensions, solutions, or emulsions in oily or aqueous vehicles, and contain formulatory agents such as suspending, stabilizing and/or dispersing agents. Alternatively, the active ingredient can be in powder form for constitution with a suitable vehicle, e.g., pyrogen free water.

In the disclosed methods, FcRn antagonists may be linked to chelators such as those described in U.S. Pat. No.5,326,856. The peptide-chelator complex may then be radiolabeled to provide an imaging agent for diagnosis or treatment of diseases or conditions involving the regulation of IgG levels.

### V. Exemplification

The present invention is further illustrated by the following examples, which should not be construed as further limiting. The contents of Sequence Listing, figures and all references, patents, and published patent applications cited throughout this application are expressly incorporated herein by reference.

### Example 1: Effect of Fc-Abdeg on serum IgG levels in cynomolgus monkeys

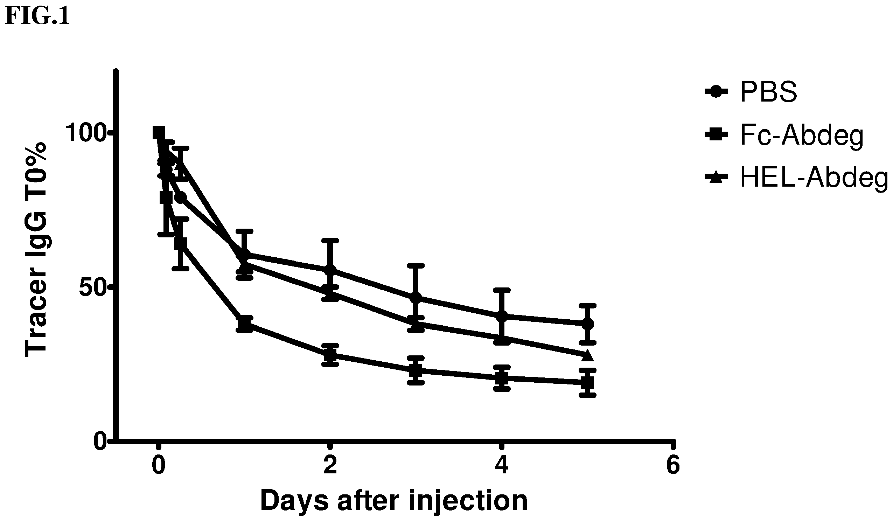

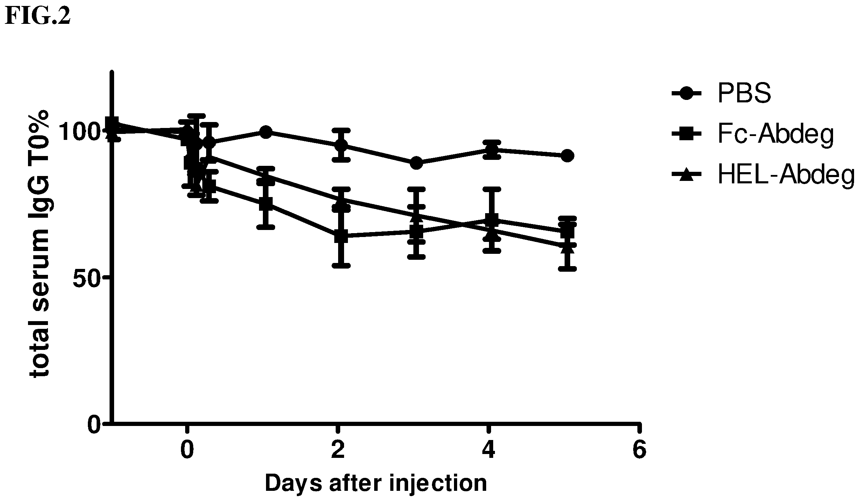

The effect of a human anti-lysozyme IgG (HEL-Abdeg) and a human IgG Fc region (Fc-Abdeg), comprising the amino acids Y, T, E, K, F, and Y at EU positions 252, 254, 256, 433, 434, and 436, respectively (Fc-Abdeg; SEQ ID NO:2), on serum IgG levels of a tracer antibody was determined in cynomolgus monkeys. Specifically, cynomolgus monkeys were administered 1mg/kg of an anti-murine CD70 hIgG1 tracer antibody (FR70-hIgG1; Oshima et al., Int Immunol 10(4): 517-26 (1998)) by i.v. bolus injection. Animals were infused 5 minutes later with either 7 mg/kg Fc-Abdeg, 20 mg/kg HEL-Abdeg, or PBS (2 monkeys per group). Infusion was performed within 1 hour and animals were administered a volume of 10 ml/kg. Blood samples (3x 150 µl) were taken at 5 min prior to dosing ("pre-dose") and 5 min, 2h, 6h, 24h, 48h, 72h, 96h and 120h after completion of the infusion. Tracer levels were determined by performing a mCD70-binding ELISA and data were plotted relative to tracer levels at end of dosing (FIG.1). Total cynomolgus IgG levels were also determined (FIG.2). The results of these experiments show that Fc-Abdeg reduced tracer antibody more efficiently than equimolar amounts of HEL-Abdeg.

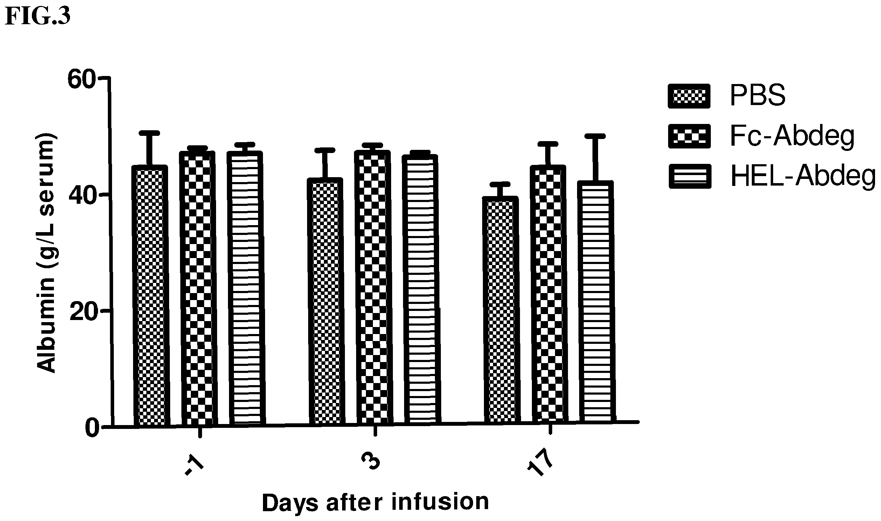

In addition to its key role in the IgG salvage pathway, FcRn is also involved in albumin homeostasis (Chaudhury et al., J Exp Med. 197(3):315-22 (2003). FcRn interacts with IgG-Fc and albumin at distinct sites and binding can happen concurrently (Andersen et al., Nat Commun. 3:610 (2012)). Conceptually, blockage of IgG recycling using Abdeg-modified molecules should not interfere with albumin-FcRn interaction. This hypothesis was confirmed in a mouse *in vivo* study, where the authors showed no influence of an Abdeg-equipped hIgG1 molecule on albumin levels (Patel et al., J Immunol 187(2): 1015-22 (2011)). In the experiment described above, albumin levels were also determined at day -3, day 3 and day 17 after the completion of the infusion. Analogous to the mouse study, no significant changes in albumin levels were observed after Fc-Abdeg or HEL-Abdeg treatment (see FIG.3).

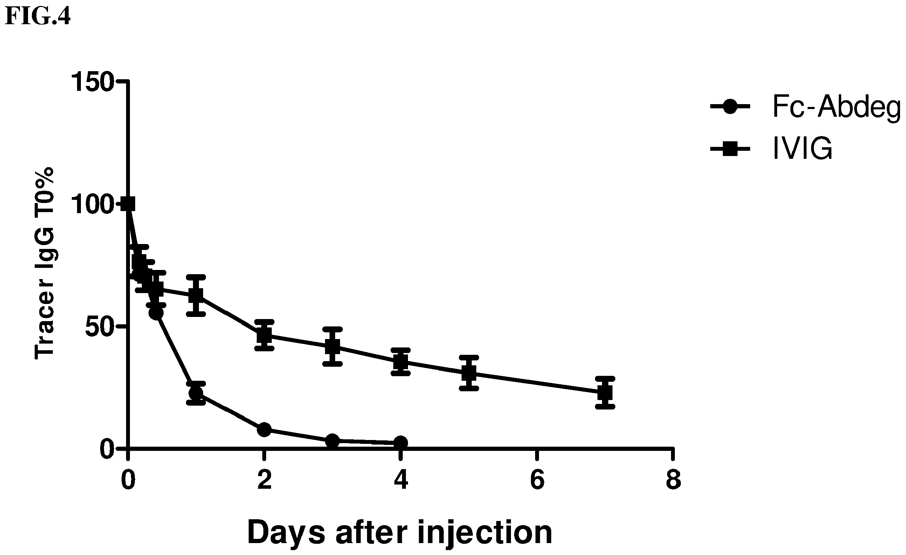

In a subsequent experiment, the antibody-depleting potency of Fc-Abdeg was compared to IVIG. Specifically, cynomolgus monkeys were administered with 1 mg/kg tracer antibody (FR70-hIgG1) 2 days prior to dosing with 70 mg/kg Fc-Abdeg or 2 g/kg IVIG (2 monkeys per group). Infusion of Fc-Abdeg and IVIG was performed within 4 hours and animals were administered a volume of 20 ml/kg. Blood (3x 150 µl) samples were taken 5 min prior to dosing ("pre-dose"), and 5 min, 2h, 6h, 24h, 48h, 72h, 96h, 120h, and 168h after completion of the infusion. Tracer levels were determined by mCD70-binding ELISA and plotted relative to pre-dose levels (FIG.4). In comparison with IVIG treatment at clinical dose (2 g/kg), 70 mg/kg Fc-Abdeg showed significantly enhanced kinetics of tracer clearance and was also able to clear more efficiently (>95% tracer clearance in 4 days for Abdeg versus -75% in 7 days for IVIG).

### Example 2: Effect of afucoslvation on Fc-Abdeg affinity for human CD16a and murine CD16-2

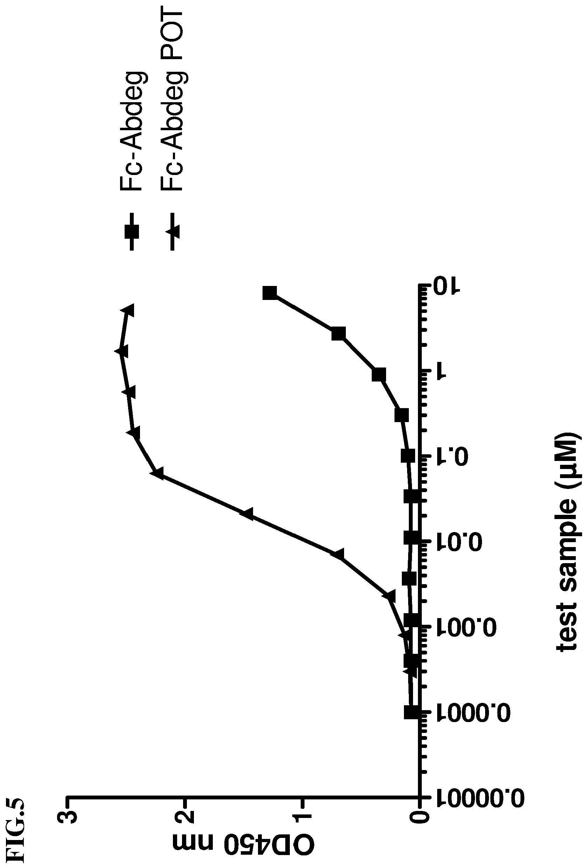

The binding affinity of Fc-Abdeg for hCD16a was determined and compared to the afucosylated form (Fc-Abdeg-POT). In the same experiment an Fc-Abdeg variant showing improved affinity for all FcyRs was included ("Fc-Abdeg-S239D/I332E). Specifically, a Maxisorp plate was coated with 100ng/well of Neutravidin Biotin-binding Protein (ThermoScientific, 31000) and incubated overnight at 4°C. The following day, the plate was blocked with PBS+1% casein for 2 hours at room temperature. Subsequently, 100µl/well of a 250ng/ml solution (dilution in PBS + 0.1% casein) of biotinylated hCD16a (Sino Biological Inc., 10389-H27H1-B) was added to the plate and incubated for 1 hour at room temperature prior to applying a concentration gradient of Fc-Abdeg or Fc-Abdeg-POT molecules (1 µM-0.005 nM) for a further hour. Binding to hCD16a was detected using an HRP-conjugated polyclonal goat anti-human Fc antibody (Jackson ImmunoResearch, 109-035-008) (incubation 1 hour at RT, dilution 1/50,000 in PBS+0.1% casein), followed by addition of 100µl room temperature-equilibrated TMB (SDT-reagents #s TMB). Plates were incubated for 10 minutes prior to addition of 100µl 0.5N H₂SO₄ and OD450nm measurement. EC50 values were determined using GraphPad Prism software. The results of these experiments, set forth in FIG.5, show that defucosylation of the Fc-Abdeg molecule results in a >30-fold increase in affinity for hCD16a (EC₅₀= 13nM for the Fc-Abdeg-POT vs. EC₅₀ >0.4µM for the fucosylated Fc-Abdeg). As expected, binding affinity of the Fc-Abdeg-S239D/I332E variant for hCD16a was increased compared to wild-type Fc-Abdeg (EC₅₀=6nM).

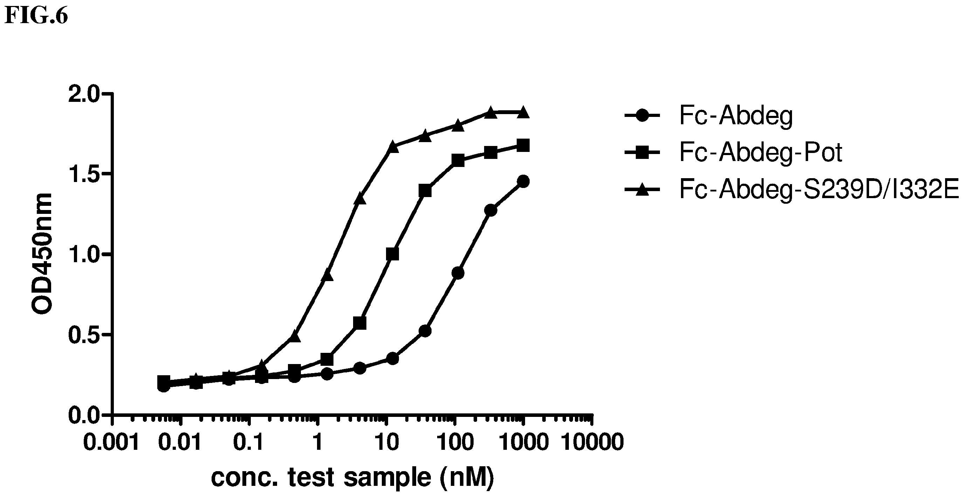

Using a similar experimental procedure as described above, the binding affinity for murine CD16-2 (Sino Biological Inc., 50036-M27H-B) was determined. The results of these experiments, set forth in FIG.6, again show an increased affinity of the afucosylated variant compared to the wild-type Fc-Abdeg (EC₅₀=11nM vs. EC₅₀> 100nM). The fold increase in affinity for mCD16-2 of the Fc-Abdeg-POT variant over the wild-type Fc-Abdeg is lower compared to that observed for binding to human CD16a. This effect was not observed for the Fc-Abdeg-S239D/I332E variant (EC₅₀=2nM), which has a similar fold increase in affinity over wild type Fc-Abdeg for both the human and murine CD16 (EC₅₀=2nM).

Autoantibodies complexed with self antigens bind to activating FcyRs and thereby trigger the autoimmune diseases, which occur in part because of immunologically mediated inflammation against self tissue. The ability of Fc-Abdeg to antagonize the interaction of autoimmune antibodies and FcγRIII receptors on NK cells was evaluated in two ADCC-based assays.

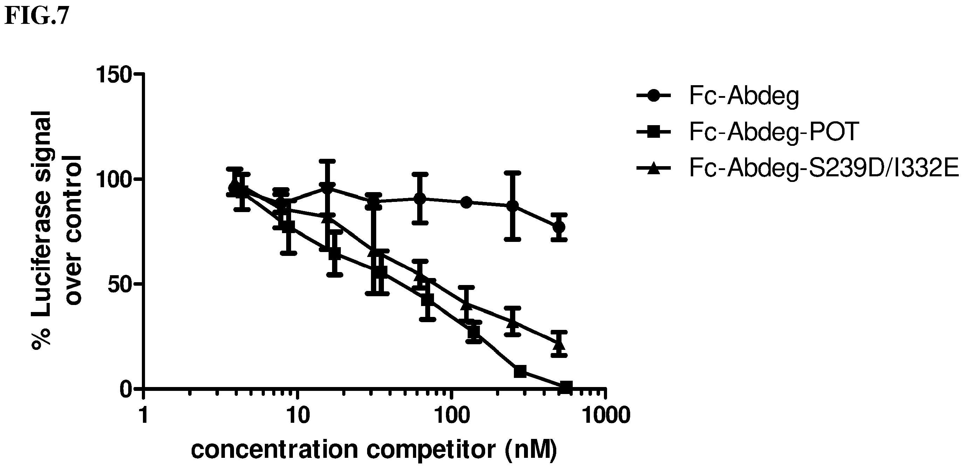

Initially, an ADCC reporter bioassay (Promega, G7016) was used to analyze the competitive hCD16a binding potency of Fc-Abdeg, Fc-Abdeg-POT and Fc-Abdeg-S239D/I332E. Specifically, 10,000 CD20-expressing Raji cells (target cells) were incubated with 60,000 Jurkat cells expressing hCD16a (effector cells) in presence of 100ng/ml anti-CD20 antibody and increasing concentration of competitor. Cells were incubated for 6 hours at 37°C prior to measuring the bioluminescence signal, which is a measure of ADCC-activity. The luciferase signal was plotted relative to the signal obtained by 100ng/ml anti-CD20 in the absence of competitor (see FIG.7). These experiments demonstrate that both Fc-Abdeg-POT and Fc-Abdeg-S239D/I332E efficiently block the anti-CD20-induced ADCC signal, whilst incubation with wild-type Fc-Abdeg does not lead to competitive binding for hCD16a expressed on Jurkat cells.

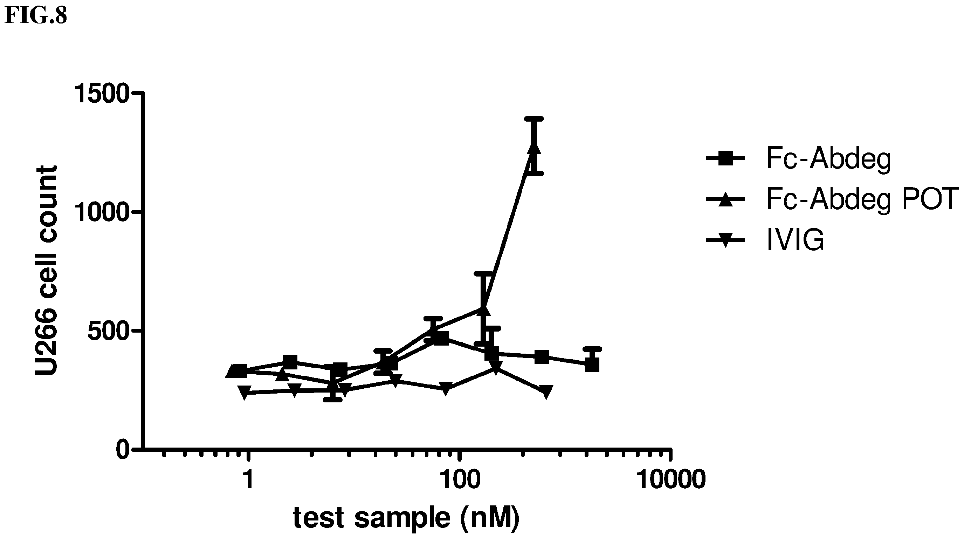

In a next ADCC assay, inhibition of the lytic activity of an anti-hCD70 antibody (27B3-hIgG1) by Fc-Abdeg and Fc-Abdeg-POT was tested as a measure of competitive hCD16 binding. Specifically, about 50,000 hCD70-expressing U266 cells were spiked into about 300,000 freshly purified PBMCs from a healthy donor in the presence of 50 ng/ml of the anti-hCD70 antibody and a concentration-gradient of Fc-Abdeg, Fc-Abdeg-POT and IVIG. The U266 cells were incubated for two days, and subsequent cell lysis was analyzed by FACS using a marker specific for the U266 cells (CD28). The results of these experiments, set forth in FIG.8, show that the anti-CD70 antibody efficiently lyses U266 cells and that this depletion could be attenuated in a dose-dependent fashion by addition of Fc-Abdeg-POT but not by wild-type Fc-Abdeg nor IVIG. These data demonstrate that Fc-Abdeg POT has enhanced competitive CD16a binding properties relative to wild-type Fc-Abdeg and IVIG.

### Example 3: Murine acute ITP model

The therapeutic potency of Fc-Abdeg, Fc-Abdeg-POT, Fc-Abdeg-S239D/I332E molecules was tested in a mouse model of acute immune thrombocytopenia. Specifically, C57BL/6 mice were treated with IVIG (20mg/animal), Fc-Abdeg (1 mg/animal), Fc-Abdeg-POT (1 mg/animal), Fc-Abdeg-S239D/I332E (1mg/animal) or saline via the intraperitoneal infusion (5 animals/group). Prior to treatment, a blood sample was withdrawn for a baseline measurement of platelet counts. One hour later, mice were treated with 5µg/animal of the anti-mouse platelet antibody MWReg30 (Nieswandt et al., Blood 94:684-93 (1999)). Platelet counts were monitored over 24 hours. Platelet counts were normalized relative to the initial counts for each mouse and platelets numbers were determined using flow cytometry via anti-CD61 staining. The results of these experiments, set forth in FIG.9, demonstrate that pretreatment with Fc-Abdeg reduces MWReg30-induced thrombocytopenia with a similar potency compared to a 7-fold higher molar dose of IVIG, and further, that blockade of FcyRs by Fc-Abdeg POT and Fc-Abdeg-S239D/I332E had a synergistic beneficial effect in this model, as seen by the improved platelet counts at the 180 and 1440 minutes time points.

### Example 4: Manufacturabilitv Fc-Abdeg

Fc-Abdeg (comprising Fc domains having SEQ ID NO:2) was produced in CHO cells (Evitria, Switzerland) by transient transfection. Following transfection, high titers of Fc-Abdeg were detected in the supernatants (between 200 and 400 mg/ml). A similar favorable production profile was seen when Fc-Abdeg was expressed from an expression construct stably integrated into the CHO GS-XCEED cell line (Lonza, Great-Britain). On average, stable transfectants yielded 3g/L and several clones were identified which produced up to 6g/L Fc-Abdeg in a 10 L stirred tank bioreactor.

The manufacturability of the Fc-Abdeg was further investigated by analysis of aggregates and degradation products following protein A-purification of the aforementioned Fc-Abdeg production runs. Specifically, 137 µg of Fc-Abdeg was loaded on a Superdex 200 10/300 GL gelfiltration column (GE Healthcare) coupled to an ÄktaPurifier chromatography system. Results of this experiment, set forth in FIG.10, showed that only a very small percentage of Fc-Abdeg aggregates was observed (-0.5%), whilst no Fc-Abdeg degradation products were detected. Additionally, applying various stress conditions (freeze-thaw, rotational or temperature stress) to the protein A-purified Fc-Abdeg did not lead to any apparent change in physicochemical and functional properties. Taken together, these data demonstrate the excellent manufacturability of the Fc-Abdeg.

### Example 5: Dose-escalation study of Fc-Abdeg in cynomolgus monkeys

A dose-escalation study of Fc-Abdeg was performed in cynomolgus monkeys to determine the onset of the pharmacodynamics effect as well as the saturating dose. To this end, cynomolgus monkeys were administered 1 mg/kg of an anti-murine CD70 hIgG1 tracer antibody (FR70-hIgG1) by i.v. bolus injection. Animals were infused 48 hours later with various doses of FC-Abdeg (0.2 mg/kg, 2 mg/kg, 20mg/kg or 200mg/kg) or vehicle (PBS). Infusion was performed within 3 hours and animals were administered a volume of 36.36 ml/kg. Each test group consisted of 2 animals. Blood samples (3x150µl) were taken 5 minutes prior to dosing ("pre-dose") and 5 min, 2h, 6h, 24h, 48h, 72h, 5 days, 7 days, 10 days and 14 days after the end of the infusion. Both tracer IgG (see Figure 11) and endogenous IgG levels (see Figure 12) were determined by ELISA and plotted relative to pre-dose levels. Data from 70mg/kg dose were superposed from experiments set forth in Figure 4 herein. Dosing animals with 0.2 mg/kg FC-Abdeg did not significantly influence the rate of tracer clearance nor does it affect endogenous IgG levels. A clear pharmacodynamic effect was seen at 2mg/kg and this effect levels out at doses starting from 20mg/kg. A single administration of Fc-Abdeg reduces cynomolgus monkey IgGs by maximally 55% within 3-4 days.

FcRn binding is restricted to the gamma subtype of immunoglobulins, and is accountable for their much longer half-life compared to other immunoglobulin subtypes. Blocking the IgG recycling function of FcRn with Fc-Abdeg should therefore not interfere with endogenous non-IgG immunoglobulin levels, a feature which was demonstrated by measuring endogenous IgA and IgM levels in the serum samples of animals treated with 200mg/kg Fc-Abdeg (see Figures 13 and 14). Together with the observation set forth in Example 1 showing that Fc-Abdeg treatment does not influence albumin levels, these data demonstrate the specific pharmacodynamic effect of Fc-Abdeg.

Next, the pharmacokinetic profile of Fc-Abdeg was determined via ELISA. Fc-Abdeg has a short half-life (estimated half-life ~1.5 days) as has been calculated from its PK profile (see Figure 5). At saturating levels, non-FcRn binding Fc-Abdeg will be cleared efficiently due to its own mode of action. In addition, the molecular size of Fc-Abdeg is close to the cutoff for renal clearance (~60kDa).

### Example 6: Repetitive dosing study of Fc-Abdeg in cynomolgus monkeys

A single infusion of Fc-Abdeg at a dose of 20mg/kg reduces endogenous IgG levels by 55% in cynomolgus monkeys in approximately 3-4 days. In a subsequent experiment, it was assayed whether repetitive dosing of Fc-Abdeg would lower these levels even more. Two strategies were followed: one group of monkeys received an infusion of the test component every 24h during the first 4 days (days 1, 2, 3, 4), whilst the other group received the drug every four days (days 1, 5, 9, 13). For each individual administration, Fc-Abdeg was administered at a dose of 20mg/kg. Each test group consisted of 2 animals. Infusion procedure was identical to the one described in the Example 1. A clear pharmacodynamic difference between the two different groups is observed as of day 7 (see Figure 16). Dosing Fc-Abdeg every day for the first 4 days showed a similar reduction in IgG levels as a single infusion at the same dose (dashed line, data from Example 1 superposed as reference). Dosing Fc-Abdeg once every four days results in a more profound and longer reduction of these levels. A maximal reduction up to 25% of initial levels is observed in this treatment group.

The pharmacokinetic profile of Fc-Abdeg was determined (see Figure 7) and corresponds to the findings from the dose-escalation study set forth in Example 5.

### Example 7: Further dose-escalation study of Fc-Abdeg in cynomolgus monkeys

A follow-up dose-escalation study of Fc-Abdeg was performed in cynomolgus monkeys to further explore the onset and saturation of the pharmacodynamics effects. To this end, animals were infused with various doses of FC-Abdeg (10 mg/kg, 30 mg/kg, 50 mg/kg and 100mg/kg) or vehicle (PBS). Infusion was performed within two hours and each cohort consisted of four animals (two males and two females). Endogenous IgG levels were determined by ELISA and plotted relative to pre-dose levels (see Figure 18). An intermediate pharmacodynamic effect was observed at the 10mg/kg dose and this effect leveled out at doses starting from 30mg/kg. The rate of IgG depletion, onset of action and pharmacokinetic profile (see Figure 19, see Table 2) were similar to the findings set forth in Example 5.

**Table 2. Pharmacokinetic properties of Fc-Abdeg in cynomolgus monkeys**

| **Non-compartment analysis of Fc-Abdeg** | | | | | | | | | | |
|---|---|---|---|---|---|---|---|---|---|---|
| **Dose [mg/kg]** | **Cmax^{#} [µg/mL]** | **tmax^{#} [h]** | **t_{1/2} [h]** | | **tₗₐₛₜ^{#} [h]** | **CL [mL/h/ kg]** | **AUC_{0-t last} [µg^{∗}h/mL]** | **AUC_{0-∞} [µg^{∗}h/mL]** | **AUC_{0-∞} /dose [µg^{∗}h^{∗}kg/** | **DPF** |
| **MALES** | | | | | | | | | | |
| **10** | 179.1 | 2.0 | 22.8 | 672.0 | | 1.95 | 5138 | 5138 | 514 | - |
| **30** | 512.3 | 2.0 | 24.7 | 672.0 | | 1.99 | 15317 | 15317 | 511 | 0.99 |
| **50** | 850.7 | 2.0 | 26.0 | 672.0 | | 1.93 | 26025 | 26025 | 521 | 1.01 |
| **100** | 1414.9 | 2.0 | 28.2 | 540.0 | | 2.28 | 43865 | 43865 | 439 | 0.85 |

| **FEMALES** | | | | | | | | | | |
|---|---|---|---|---|---|---|---|---|---|---|
| **10** | 188.3 | 2.0 | 21.3 | 672.0 | | 1.80 | 5573 | 5573 | 557 | - |
| **30** | 447.6 | 2.0 | 34.5 | 672.0 | | 2.13 | 14094 | 14094 | 470 | 0.84 |
| **50** | 978.9 | 2.0 | 27.5 | 456.0 | | 1.78 | 28101 | 28104 | 562 | 1.01 |
| **100** | 1586.8 | 2.0 | 28.1 | 576.0 | | 2.02 | 49553 | 49553 | 496 | 0.89 |

| | | | | | | | | | | |
|---|---|---|---|---|---|---|---|---|---|---|
| #: Values obtained from serum analysis of Fc-Abdeg, all other values calculated by toxicokinetic analysis. -: not computable or no reasonable interpretation from the available data DPF: Dose proportion factor = [AUC_{0-t last} (x mg/kg)/ AUC_{0-t last} (10 mg/kg)\|/ [(x mg/kg)/ (10 mg/kg)] | | | | | | | | | | |

### Example 8: Further repetitive dosing study of Fc-Abdeg in cynomolgus monkeys

A follow-up repetitive dosing study of Fc-Abdeg was performed in cynomolgus monkeys to further examine the pharmacodynamic effect of chronic Fc-Abdeg administration. To this end, animals were infused every 48h with various doses of FC-Abdeg (3 mg/kg, 30 mg/kg and 100mg/kg) or vehicle (PBS) for a total of 15 infusions. After the treatment period a recovery period of 30 days was included. Each cohort consisted of four animals, with the exception of the 100 mg/kg cohort (which consisted of three animals). Endogenous IgG levels were determined by ELISA and plotted relative to pre-dose levels (see Figure 20, average ± SEM). A clear drop in IgG levels was observed for all tested doses, and this pharmacodynamic effect persisted until the end of the treatment period. After the treatment period, IgG levels were restored to baseline within 10 days. The pharmacokinetic profile is shown in Figure 21.

### Example 9: Chronic dosing study of Fc-Abdeg in cynomolgus monkeys

Cynomolgus monkeys were infused with an i.v. loading dose of 20mg/kg FC-Abdeg and further received daily subcutaneous administration of Fc-Abdeg at 1, 3, or 5 mg/kg beginning 24 hours after the initial dose and continuing for for 12 days. Each test group consisted of two animals. IgG levels were determined by ELISA and plotted relative to pre-dose levels (see Figure 22). Data is also presented for a single cynomolgus monkey from the 3 mg/kg cohort in which the treatment persisted beyond 12 days. This monkey was infused with an i.v. loading dose of 20mg/kg FC-Abdeg, received daily subcutaneous administration of Fc-Abdeg at 3 mg/kg beginning 24 hours after the initial dose and continuing for 28 days, and was followed for a further treatment-free period of 32 days (see Figure 23). IgG levels were reduced up to 60% of original levels and remained consistently low throughout the treatment period. After the treatment period, IgG levels restored to baseline levels within two weeks.

The invention will now be understood with reference to the following clauses:
1. A method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject in a dose of between about 0.2 and about 200 mg/kg.
2. A method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject at least twice in 20 days.
3. The method of clause 2, wherein the FcRn-antagonist is administered to the subject at a frequency of once every 1, 2, 3, 4, 5, 6, 7, 8, 9 or 10 days.
4. The method of clause 2, wherein the FcRn-antagonist is administered to the subject at a frequency of once every 4 days.
5. The method of clause 2, wherein the FcRn-antagonist is administered to the subject at a frequency of once every 7 days.
6. The method of clause 2 or 3, wherein the FcRn-antagonist is administered to the subject 2, 3, 4, 5, 6, 7, 8, 9, 10, 11, 12, 13, 14, 15, 16, 17, 18, 19, or 20 times in 20 days.
7. A method of reducing the serum levels of an Fc-containing agent in a subject, the method comprising administering to the subject an effective amount of an isolated FcRn-antagonist comprising a variant Fc region, or FcRn-binding fragment thereof, wherein the Fc domains of the variant Fc region comprise the amino acids Y, T, E, K, F, and Y at EU positions 252, 254, 256, 433, 434, and 436 respectively, and wherein the FcRn-antagonist is administered to the subject at a frequency of once every 48 hours for four weeks.
8. The method of any one of clauses 2-6, wherein the FcRn-antagonist is administered to the subject in a dose of between about 0.2 and about 200 mg/kg.
9. The method of any one of the preceding clauses, wherein the FcRn-antagonist is administered to the subject in a dose of about 0.2, 1, 2, 3, 5, 10, 20, 25, 30, 50, 70, 100, or 200 mg/kg.
10. The method of any one of the preceding clauses, wherein the FcRn-antagonist is administered to the subject in a dose of about 10 mg/kg.
11. The method of any one of the preceding clauses, wherein the FcRn-antagonist is administered to the subject in a dose of about 20 mg/kg.
12. The method of any one of the preceding clauses, wherein the FcRn-antagonist is administered to the subject in a dose of about 25 mg/kg.
13. The method of any one of the preceding clauses, wherein the FcRn-antagonist is administered intravenously.
14. The method of any one of the preceding clauses, wherein the FcRn-antagonist is administered subcutaneously.
15. The method of any one of the preceding clauses, wherein the FcRn-antagonist is administered to the subject in more than one dose, wherein the first dose administered to the subject is administered intravenously, and wherein one or more of the subsequent doses are administered subcutaneously.
16. The method of any one of the preceding clauses, wherein the FcRn-antagonist does not comprise an antibody variable region.
17. The method of any one of the preceding clauses, wherein the FcRn-antagonist does not comprise a CH1 domain.
18. The method of any one of the preceding clauses, wherein the FcRn-antagonist does not comprise a free cysteine residue.
19. The method of any one of the preceding clauses, wherein the variant Fc region is an IgG Fc region.
20. The method of any one of the preceding clauses, wherein the variant Fc region is an IgG1 Fc region.
21. The method of any one of the preceding clauses, wherein the amino acid sequence of the Fc domains of the variant Fc region comprises the amino acid sequence set forth in SEQ ID NO:1, 2, or 3.
22. The method of any one of the preceding clauses, wherein the amino acid sequence of the Fc domains of the variant Fc region consists of the amino acid sequence set forth in SEQ ID NO:1.
23. The method of any one of the preceding clauses, wherein the FcRn-antagonist consists of a variant Fc region, wherein the amino acid sequence of the Fc domains of the variant Fc region consists of the amino acid sequence set forth in SEQ ID NO:1, 2, or 3.
24. The method of any one of the preceding clauses, wherein the variant Fc region has an increased affinity for an Fc gamma receptor relative to the affinity of a wild-type IgG1 Fc region for the Fc gamma receptor.
25. The method of any one of the preceding clauses, wherein the variant Fc region has increased affinity for CD16a.
26. The method of any one of the preceding clauses, wherein the Fc domains of the variant Fc region do not comprise an N-linked glycan at EU position 297.
27. The method of any one of clauses 1-25, wherein the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region, or FcRn-binding fragment thereof, comprising an afucosylated N-linked glycan at EU position 297.
28. The method of any one of clauses 1-25, wherein the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region or FcRn-binding fragment thereof, comprising an N-linked glycan having a bisecting GlcNac at EU position 297.
29. The method of any one of the preceding clauses, wherein the variant Fc region is linked to a half-life extender.
30. The method of any one of the preceding clauses, wherein the half-life extender is polyethylene glycol or human serum albumin.
31. The method of any one of the preceding clauses, wherein the Fc-containing agent is an antibody or immunoadhesin.
32. The method of any one of the preceding clauses, wherein the Fc-containing agent is a therapeutic or diagnostic agent.
33. The method of any one of the preceding clauses, wherein the Fc-containing agent is an imaging agent.
34. The method of any one of the preceding clauses, wherein the Fc-containing agent is an antibody drug conjugate.
35. The method of any one of the preceding clauses, wherein the Fc-containing agent is a pathogenic antibody.
36. The method of any one of the preceding clauses, wherein the Fc-containing agent is an autoantibody.
37. The method of any one of the preceding clauses, wherein the subject has an antibody-mediated disease or disorder, and wherein administration of the FcRn-antagonist to the subject ameliorates the disease or disorder.
38. The method of clause 37, wherein the antibody-mediated disease or disorder is an autoimmune disease.
39. The method of clause 38, wherein the autoimmune disease is selected from the group consisting of allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, dilated cardiomyopathy, discoid lupus, epidermolysis bullosa acquisita, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic membranous neuropathy, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lichen sclerosus, lupus erthematosis, Meniere's disease, mixed connective tissue disease, mucous membrane pemphigoid, multiple sclerosis, type 1 diabetes mellitus, Multifocal motor neuropathy (MMN), myasthenia gravis, paraneoplastic bullous pemphigoid, pemphigoid gestationis, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, relapsing polychondritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.
40. The method of clause 37, wherein the disease or disorder is treatable using intravenous immunoglobulin (IVIG), plasmapheresis and/or immunoadsorption.
41. The method of clause 38, wherein the autoimmune disease is an autoimmune channelopathy.
42. The method of clause 41, wherein the channelopathy is selected from the group consisting of autoimmune limbic encephalitis, epilepsy, neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, neuromyotonia, pediatric autoimmune neuropychiatric disorders associated with streptococcal infection (PANDAS), and Glycine receptor antibody-associated disorder.
43. The method of clause 37, wherein the antibody-mediated disease or disorder is hyperglobulinemia.
44. The method of any one of the preceding clauses, wherein the FcRn antagonist is administered to the subject simultaneously or sequentially with an additional therapeutic agent.
45. The method of clause 44, wherein the additional therapeutic agent is an anti-inflammatory agent.
46. The method of clause 44, wherein the additional therapeutic agent is a leucocyte depleting agent.
47. The method of clause 45, wherein the leucocyte depleting agent is a B-cell depleting agent.
48. The method of clause 47, wherein the B-cell depleting agent is an antibody.
49. The method of clause 48, wherein the B-cell depleting agent is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86.
50. The method of clause 49, wherein the additional therapeutic agent is rituximab, daclizumab, basiliximab, muronomab-CD3, infliximab, adalimumab, omalizumab, efalizumab, natalizumab, tocilizumab, eculizumab, golimumab, canakinumab, ustekinumab, belimumab, or a combination thereof.
51. The method of any one of the preceding clauses, wherein the subject is a human or cynomolgus monkey.

## Claims

1. An isolated FcRn-antagonist consisting of a variant IgG1 Fc region for use in a method of treating an autoimmune disease in a subject, the method comprising administering to the subject an effective amount of the isolated FcRn-antagonist consisting of the variant IgG1 Fc region, wherein the Fc domains of the variant Fc region comprise the amino acids Y, T, E, K, F, and Y at EU positions 252, 254, 256, 433, 434, and 436 respectively,
wherein the FcRn-antagonist is administered to the subject 2 times in 20 days, and wherein the FcRn-antagonist is administered to the subject in a dose of between 2 and 200 mg/kg.

2. The isolated FcRn-antagonist for use according to claim 1, wherein the amino acid sequence of the Fc domains of the variant Fc region consists of the amino acid sequence set forth in SEQ ID NO: 1, 2, or 3.

3. The isolated FcRn-antagonist for use according to claim 1 or claim 2, wherein:
(i) the variant Fc region has an increased affinity for an Fc gamma receptor relative to the affinity of a wild-type IgG1 Fc region for the Fc gamma receptor; or
(ii) the variant Fc region has increased affinity for CD16a.

4. The isolated FcRn-antagonist for use according to any one of claims 1-3, wherein the Fc domains of the variant Fc region do not comprise an N-linked glycan at EU position 297.

5. The isolated FcRn-antagonist for use according to any one of claims 1-3, wherein:
(i) the FcRn-antagonist is provided as a composition comprising a plurality of FcRn-antagonist molecules, wherein at least 50% (optionally, at least 60, 70, 80, 90, 95, or 99%) of the plurality of FcRn-antagonist molecules consist of the variant Fc region additionally comprising an afucosylated N-linked glycan at EU position 297; or
(ii) the FcRn-antagonist is provided as a composition comprising a plurality of FcRn-antagonist molecules, wherein at least 50% (optionally, at least 60, 70, 80, 90, 95, or 99%) of the plurality of FcRn-antagonist molecules consist of the variant Fc region additionally comprising an N-linked glycan having a bisecting GlcNac at EU position 297.

6. The isolated FcRn-antagonist for use according to any one of claims 1-5, wherein the FcRn-antagonist is administered intravenously.

7. The isolated FcRn-antagonist for use according to any one of claims 1-6, wherein the FcRn-antagonist is administered to the subject at a frequency of once every 10 days.

8. The isolated FcRn-antagonist for use according to any one of the claims 1-7, wherein the FcRn-antagonist is linked to a half-life extender, optionally wherein the half-life extender is polyethylene glycol or human serum albumin.

9. The isolated FcRn-antagonist for use according to any one of the claims 1-8, wherein the autoimmune disease is selected from the group consisting of allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, dilated cardiomyopathy, discoid lupus, epidermolysis bullosa acquisita, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic membranous neuropathy, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lichen sclerosus, lupus erythematosis, Meniere's disease, mixed connective tissue disease, mucous membrane pemphigoid, multiple sclerosis, type 1 diabetes mellitus, Multifocal motor neuropathy (MMN), myasthenia gravis, paraneoplastic bullous pemphigoid, pemphigoid gestationis, pemphigus vulgaris, pemphigus foliaceus, pernicious anemia, polyarteritis nodosa, polychrondritis, polyglandular syndromes, polymyalgia rheumatica, polymyositis and
dermatomyositis, primary agammaglobinulinemia, primary biliary cirrhosis, psoriasis, psoriatic arthritis, relapsing polychondritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteritis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

10. The isolated FcRn-antagonist for use according to claim 9, wherein the autoimmune disease is an autoimmune channelopathy, optionally wherein the channelopathy is selected from the group consisting of autoimmune limbic encephalitis, epilepsy, neuromyelitis optica, Lambert-Eaton myasthenic syndrome, myasthenia gravis, anti-N-Methyl-D-aspartate (NMDA) receptor encephalitis, anti-α-Amino-3-hydroxy-5-methyl-4-isoxazolepropionic acid (AMPA) receptor encephalitis, Morvan syndrome, neuromyotonia, pediatric autoimmune neuropychiatric disorders associated with streptococcal infection (PANDAS), and Glycine receptor antibody-associated disorder.

11. The isolated FcRn-antagonist for use according to any one of claims 1-10, wherein the FcRn-antagonist is administered to the subject simultaneously or sequentially with an additional therapeutic agent.

12. The isolated FcRn-antagonist for use according to claim 11, wherein the additional therapeutic agent is an anti-inflammatory agent.

13. The isolated FcRn-antagonist for use according to claim 11, wherein the additional therapeutic agent is a leukocyte depleting agent, optionally wherein the leukocyte depleting agent is a B-cell depleting agent.

14. The isolated FcRn-antagonist for use according to claim 13, wherein the B-cell depleting agent is an antibody, optionally wherein the B-cell depleting agent is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86, further optionally wherein the additional therapeutic agent is rituximab, daclizumab, basiliximab, muronomab-CD3, infliximab, adalimumab, omalizumab, efalizumab, natalizumab, tocilizumab, eculizumab, golimumab, canakinumab, ustekinumab, belimumab, or a combination thereof.

15. The isolated FcRn-antagonist for use according to any one of claims 1-14, wherein the subject is a human or cynomolgus monkey.


SEQUENCE LISTING

<110>  ARGENX BVBA

<120>  METHODS OF REDUCING SERUM LEVELS OF FC-CONTAINING AGENTS USING
       FCRN ANTAGONISTS

<130>  NLW/NTR/P139435EP01

<150>  62/130,076
<151>  2015-03-09

<160>  3

<170>  PatentIn version 3.5

<210>  1
<211>  221
<212>  PRT
<213>  Homo sapiens

<220>
<223>  Fc region

<400>  1

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Tyr Ile Thr Arg Glu Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly

145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu Lys
        195                 200                 205

Phe His Tyr Thr Gln Lys Ser Leu Ser Leu Ser Pro Gly
    210                 215                 220

<210>  2
<211>  227
<212>  PRT
<213>  Homo sapiens

<220>
<223>  Fc region

<400>  2

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Tyr
            20                  25                  30

Ile Thr Arg Glu Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu Lys Phe His Tyr Thr Gln Lys Ser Leu Ser Leu Ser
    210                 215                 220

Pro Gly Lys
225

<210>  3
<211>  226
<212>  PRT
<213>  Homo sapiens

<220>
<223>  Fc region

<400>  3

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Tyr
            20                  25                  30

Ile Thr Arg Glu Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu Lys Phe His Tyr Thr Gln Lys Ser Leu Ser Leu Ser
    210                 215                 220

Pro Gly
225